# EUROPEAN PATENT APPLICATION

(11) **EP 2 613 154 A2**
(43) Date of publication of application: **10.07.2013**
(21) Application number: 13157657.1
(22) Date of filing: 07.06.2006
(51) Int. Cl.: G01N 33/68, C12Q 1/48

(54) **Process for the identification of novel enzyme interacting compounds**

(30) Priority: 14.06.2005 EP 05012722; 25.08.2005 US 711399 P; 14.03.2006 US 782170 P
(62) Divisional of application: 06763568.0
(71) Applicant: Cellzome GmbH, 69117 Heidelberg (DE)
(72) Inventor: Drewes, Gerard, 69121 Heidelberg (DE); Küster, Bernhard, Dr., 85417 Marzling (DE); Kruse, Ulrich, Dr., 69221 Dossenheim (DE); Hopf, Carsten, Dr., 68165 Mannheim (DE); Eberhard, Dirk, 69256 Mauer (DE); Bantscheff, Marcus, 69234 Dielheim (DE); Raida, Manfred, 259281 Singapore (SG); Reader, Valerie, CB21 4HU Linton Cambridge (GB); Middlemiss, David, CM23 3NF Bishops Stortford Hertfordshire (GB)
(74) Representative: Povey, Alexander W.G.

(57) **Abstract**

The present invention relates to methods for the characterization of enzymes or of enzyme-compound complexes, wherein the enzyme is obtained from a protein preparation with the help of at least one broad spectrum ligand immobilized on a solid support and wherein the enzyme is characterized by mass spectrometry. These methods are useful for the screening of non-immobilized compound libraries, selectivity profiling of lead compounds and mechanism of action studies in living cells.

## Description

The present invention relates to methods for the characterization of enzymes or enzyme-compound complexes using enzyme ligands bound to solid supports.

The goal of drug discovery is to develop effective and safe medicines. In order to achieve this goal, pharmaceutical research aims at identifying preferably small molecule drugs directed at drug targets that are known to be causative for the disease of interest.

Traditionally, the majority of small molecule drugs are directed against receptor proteins (cell membrane receptors such as G protein coupled receptors (GPCRs) or nuclear hormone receptors), ion channels and enzymes. Of the enzymes, of particular interest are e.g. proteases, phosphodiesterases and kinases (Review: Drews, 2000, "Drug discovery: a historical perspective", Science 287, 1960-1964).

Proteases are considered as tractable drug targets as demonstrated by the effective management of AIDS with HIV protease inhibitors or the use of angiotensin-converting enzyme inhibitors to treat hypertension. For the treatment of cancer protease inhibitors directed against matrix metalloproteinases and caspases are under development (Docherty et al., 2003, "Proteases as drug targets", Biochemical Society Symposia 70, 147-161).

Phosphodiesterases (PDEs) comprise a family of enzymes that catalyse the hydrolysis of cAMP or cGMP and are implicated in various diseases. The PDE5 inhibitor sildenafil (Viagra) provides an effective treatment for erectile dysfunction. Currently PDE4 inhibitors (e.g. cilomast and roflumast) are in clinical testing as anti-inflammatory therapeutics. A major challenge in this field is the development of PDE isotype specific inhibitors in order to avoid cross-reactivity that is responsible for side effects (Card et al., 2004, "Structural basis for the activity of drugs that inhibit phosphodiesterases", Structure 12, 2233-2247).

Kinases catalyse the phosphorylation of proteins, lipids, sugars, nucleosides and other cellular metabolites and play key roles in all aspects of eukaryotic cell physiology. Phosphorylation of proteins is a common posttranslational modification of proteins and affects protein structure and function in numerous ways.

One kinase class that has become a recent focus of drug discovery comprises the protein kinases because they were shown to play important roles in the initiation and progression of tumors through dysregulation of signal transduction pathways (EGF receptor in lung cancer; overexpression of the ErbB2/Her-2 receptor in breast cancer; BCR-ABL fusion protein in leukemia; Review: Blume-Jensen and Hunter, 2001, "Oncogenic kinase signaling", Nature 411, 355-365).

The complement of protein kinases encoded in the human genome comprises 518 family members (kinome) which can be grouped into several subfamilies according to sequence similarity (Review: Manning et al., 2002, The Protein Kinase Complement of the Human Genome, Science 298, 1912-1934). In any given cell or tissue only a subset of the kinome is expressed. Kinases transfer phosphate groups from ATP to substrate molecules and thereby influence the stability, activity and function of their targets. The ATP binding pocket of different kinases is structurally similar and therefore it is considered difficult to develop selective ATP-competitive inhibitors.

The kinase family is a very large enzyme family (compared to other enzyme classes relevant as drug targets, e.g. phosphodiesterases) providing multiple opportunities for drug discovery but also unique challenges (large size of family; structural similarity of the ATP binding pockets; high intracellular ATP concentration) (Review: Cohen, P., 2002, Protein kinases - the major drug targets of the twenty-first century? Nature Reviews Drug Discovery, volume 1, 309-315).

Another kinase class of interest are lipid kinases. Lipid kinases catalyse the transfer of gamma-phosphate groups from nucleoside triphosphates to lipid substrates.

Lipid kinases such as the phosphoinositide 3-kinase (PI3K) family members are known to be modulators of the cellular response to growth factors, hormones and neurotransmitters and are involved in cancer, diabetes and other diseases (Fruman et al., 1998. Phosphoinositide kinases. Annual Review Biochemistry 67, 481-507; Cantley, L.C., 2002, Science 296, 1655-1657).

One prerequiste for the identification of compounds interacting with proteins, e.g. enzymes, is the provision of protein preparations containing as many proteins as possible of one class in a great purity. Especially, the provision of many proteins of one class (e.g. kinases) is important since this enables the screening of potentially pharmaceutically interesting compounds against many members of the protein family (so called hit identification). Other feasible uses of such protein preparations include the testing of chemically optimized compounds (lead optimization), the determination of the selectivity of a given compound (selectivity profiling) as well as the confirmation of the mode of action of a given compound.

In the art, several strategies have been proposed to assess this issue.

One approach to enrich ATP-binding proteins such as kinases and other nucleotide-binding proteins from cell extracts relies on immobilized ATP as affinity reagent, i.e. on the use of a ligand binding potentially all ATP-binding enzymes. In this case ATP is covalently immobilized by coupling the gamma-phosphate group through a linker to a resin (Graves et al., 2002, Molecular Pharmacology 62, No. 6 1364-1372; US 5536822). This approach was further extended to coupling single compounds of combinatorial compound libraries (WO00/63694).

One disadvantages of immobilized ATP is that the affinity for kinases is rather low leading to inefficient capturing of kinases or rapid elution due to high off-rates. Another disadvantage is that kinases are not preferentially captured, but also other classes of ATP-binding proteins which can be expressed at much higher levels in the cell. The more abundant ATP-binding proteins can cause inefficient capturing due to competition or can lead to problems during the mass spectrometry analysis of the bound proteins if the analytical depth is not sufficient.

Another approach described in the art is the use of ligands specific for an individual enzyme, namely high affinity and highly selective kinase inhibitors or close derivatives (e.g. optimized drugs). These are used to enrich kinases from cell lysates and the same non-modified compound is used for specific elution in order to identify the cellular drug target or targets (Godl et al., Proc. Natl. Acad. Sci. 100, 15434-15439; WO 2004/013633). This approach is only successful if the structure-affinity-relationship (SAR) is not destroyed through the chemical modification of the drug but fails if the SAR is impaired. In addition, it is difficult to identify targets mediating unwanted side effects because the SAR of the cognate drug target and the side-effect-target can be different and the latter SAR is usually not known.

Another strategy is the in vitro expression of enzymes of a given class, e.g. kinases. Fabian and colleagues (Fabian et al., 2005, Nature Biotechnology 23(3), 329-336; WO 03084981) describe a kinase profiling method that does not rely on capturing the endogenous kinases contained in cell lysates but uses kinases displayed on bacteriophage T7. The kinases (or kinase domains) used in this assay are fusion proteins that are tagged in order to allow expression, purification and detection. In the competition binding assay these phage-tagged kinases are bound to an immobilized kinase inhibitor, treated with a non-immobilized test compound and the bound tagged kinases are quantified by real-time PCR using the phage DNA as a template. A disadvantage of this method is that the kinases need to be cloned and only a fraction of the phage-tagged kinases are folded in the correct native state. Furthermore, such protein preparations do not reflect at all the natural situation in a cell.

Yet another approach uses active-site directed probes (socalled activity-based probes) that form covalent links with target enzymes. This method was used to profile the expression of serine hydrolases with highly selective probes (Liu et al., 1999, Proc. Natl. Acad. Sci. 96, 14694-14699, WO 01/77668) and further expanded to other enzyme families by using more promiscuous probes consisting of non-directed activity-based probe libraries of rhodamine- and biotin-tagged fluorescent sulfonate esters (Adam et al., 2002, Nature Biotechnology 20, 805-809, WO 01/77684, WO 03/047509). However, it remains unclear what structural and/or catalytic properties are shared by these sulfonate-targeted enzymes. Another limitation of this approach is the difficulty to distinguish specific interactions with enzymes and non-specfic interactions caused by the intrinsic reactivity of the probes.

Finally, it was tried to enrich proteins phosphorylated on tyrosines by tyrosine specific antibodies (Blogoev et al., 2004, Nature Biotechnology 9, 1139-1145). Blogoev and colleagues describe a method that can be used to study the effect of compounds such as epidermal growth factor (EGF) on phosphotyrosine-dependent signal transduction pathways. After lysis of the EGF-stimulated cells phosphotyrosine-containing proteins are enriched by immunoprecipitation with antibodies directed against phosphotyrosine. In the second step these enriched proteins are analysed and identified by mass spectrometric analysis. One major limitation of this approach is that only proteins phosphorylated on tyrosine can be captured.

In view of this, there is a need for improved methods for the characterization of those enzymes of a given class which are expressed in a cell. Furthermore, there is a need for improved methods for the identification of enzymes being binding partners of a given compound.

The present invention satisfies these needs. In the context of the present invention, it has been surprisingly found that the use of at least one broad spectrum enzyme ligand immobilized on a solid support enables the effective isolation of enzymes out of a protein preparation, preferably a cell lysate. After this isolation, effective methods can be applied either for the characterization of the enzyme bound to the broad spectrum enzyme ligand or for the identification of compound enzyme interactions. The enzyme is preferably identified by mass spectrometry. Therefore, the present invention provides effective methods for either the characterization of enzymes or the identification of binding partners to a given compound.

In a first aspect, the present invention provides a method for the characterization of at least one enzyme, comprising the steps of:
a) providing a protein preparation containing the enzyme, preferably by harvesting at least one cell containing the enzyme and lysing the cell,
b) contacting the protein preparation under essentially physiological conditions with at least one broad spectrum enzyme ligand immobilized on a solid support under conditions allowing the binding of the enzyme to said broad spectrum enzyme ligand,
c) eluting the enzyme, and
d) characterizing the eluted enzyme by mass spectrometry.

In a second aspect, the present invention provides a method for the characterization of at least one enzyme, comprising the steps of:
a) providing two aliquots comprising each at least one cell containing the enzyme,
b) incubating one aliquot with a given compound,
c) harvesting the cells,
d) lysing the cells,
e) contacting the protein preparation under essentially physiological conditions with at least one broad spectrum enzyme ligand immobilized on a solid support under conditions allowing the binding of the enzyme to said broad spectrum enzyme ligand,
f) eluting the enzyme or enzymes, and
g) characterizing the eluted enzyme or enzymes by mass spectrometry.

According to a third aspect of the invention, the invention provides a method for the characterization of at least one enzyme, comprising the steps of:
a) providing two aliquots of a protein preparation containing the enzyme, preferably by harvesting at least one cell containing the enzyme and lysing the cell,
b) contacting one aliquot under essentially physiological conditions with at least one broad spectrum enzyme ligand immobilized on a solid support under conditions allowing the binding of the enzyme to said broad spectrum enzyme ligand,
c) contacting the other aliquot under essentially physiological conditions with at least one broad spectrum enzyme ligand immobilized on a solid support under conditions allowing the binding of the enzyme to said broad spectrum enzyme ligand and with a given compound,
d) eluting the enzyme or enzymes, and
e) characterizing the eluted enzyme or enzymes by mass spectrometry.

According to a fourth aspect of the present invention, a method for the characterization of an enzyme-compound complex is provided, comprising the steps of:
a) providing a protein preparation containing the enzyme, preferably by harvesting at least one cell containing the enzyme and lysing the cell,
b) contacting the protein preparation under essentially physiological conditions with at least one broad spectrum enzyme ligand immobilized on a solid support under conditions allowing the binding of the enzyme to said broad spectrum enzyme ligand,
c) contacting the bound enzymes with a compound to release at least one bound enzyme, and
d) characterizing the released enzyme or enzymes by mass spectrometry, or
e) eluting the enzyme or enzymes from the ligand and characterizing the enzyme or enzymes by mass spectrometry, thereby identifying one or more binding partners of the compound.

The approaches as mentioned above, especially the method of the invention according to the 4^{th} aspect, have the following advantages:
- No in vitro expression of enzymes is necessary, but endogenous enzymes from cell lysates are used.
- Surprisingly, it was found that broad specificity kinase ligands capture kinases very efficiently.
- The test compounds do not need to be linked or immobilized (label-free assay method).

The competition binding or elution is possible with any compound of interest (non-modified, non-immobilized).
- No enzyme substrates are necessary (as in biochemical enzyme assays).
- It is possible to characterize the in vivo effect of the compound on a signal transduction pathway (see 2^{nd} aspect).
- The identification of direct and indirect targets is possible.

Throughout the invention, the term "enzyme" includes also every protein or peptide in the cell being able to bind a ligand such as transporters, ion channels and proteins that interact with enzymes such as adapter proteins containing peptide interaction domains (e.g. SH2, SH3, and PDZ domains).

Preferably, however, the term "enzyme" is interpreted in its usual way as being a biocatalysator in a cell.

Throughout the invention, the term "broad spectrum enzyme ligand" refers to a ligand which is able to bind some, but not all enzymes present in a protein preparation.

The present invention preferably relates to methods for the characterization of enzymes or the identification of binding partners to a given compound, wherein the enzyme or the binding partners are included in a cell lysate. However, the methods of the present invention can also be performed with any protein preparation as a starting material, as long as the protein(s) is/are solubilized in the preparation. Examples include a liquid mixture of several proteins, a partial cell lysate which contains not all proteins present in the original cell or a combination of several cell lysates.

Partial cell lysates can be obtained by isolating cell organelles (e.g. nucleus, mitochondria, ribosomes, golgi etc.) first and then prepare protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein samples can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

Throughout the invention, the term "solid support" relates to every undissolved support being able to immobilize said broad spectrum enzyme ligand on its surface.

The method of the invention according to the second aspect encompasses as initial steps a provision of two aliquots comprising each at least one cell containing the enzyme and incubating one aliquot with a given compound. In a preferred embodiment, the at least one cell is part of the cell culture system which is divided into at least two aliquots. One aliquot of cells is then incubated with the given compound. Methods for the incubation of cell culture systems with compounds are known in the art (Giuliano et al., 2004, "High-content screening with siRNA optimizes a cell biological approach to drug discovery: defining the role of p53 activation in the cellular response to anticancer drugs". Journal of Biomolecular Screening 9(7), 557-568).

However, it is also included within the present invention that the at least one cell of each aliquot is part of an in vivo system, e. g. a mouse system or a lower vertebrate system.

For example whole embryo lysates derived from defined development stages or adult stages of model organisms such as C. elegans can be used. In addition, whole organs such as heart dissected from mice can be the source of protein preparations. These organs can also be perfused in vitro and so be treated with the test compound or drug of interest.

All methods of the present invention include at least in a preferred embodiment the steps of harvesting at least one cell containing the enzyme and lysing the cell.

In a preferred embodiment, the cell is part of a cell culture system and methods for the harvest of a cell out of a cell culture system are known in the art (literature supra).

The choice of the cell will mainly depend on the class of enzymes supposed to be analyzed, since it has to be ensured that the class of enzyme is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given class of enzymes is present in the cell.

The choice of the cell will also be influenced by the purpose of the study. If the in vivo target for a given drug needs to be identified then cells or tissues will be selected in which the desired therapeutic effect occurs (e.g. breast cancer tissue for anticancer drugs). By contrast, for the elucidation of protein targets mediating unwanted side effects the cell or tissue will be analysed in which the side effect is observed (e.g. brain tissue for CNS side effects).

Furthermore, it is envisaged within the present invention that the cell containing the enzyme may be obtained from an organism, e. g. by biopsy. Corresponding methods are known in the art. For example, a biopsy is a diagnostic procedure used to obtain a small amount of tissue, which can then be examined miscroscopically or with biochemical methods. Biopsies are important to diagnose, classify and stage a disease, but also to evaluate and monitor drug treatment. Breast cancer biopsies were previously performed as surgical procedures, but today needle biopsies are preferred (Oyama et al., 2004, Breast Cancer 11(4), 339-342).

The described methods of the invention allow to profile the tissue samples for the presence of enzyme classes. For example, mutated enzymes causative for the disease can be identified (e.g. point mutations that activate oncogenic kinases). In addition, mutated enzymes that arise during treatment and are responsible for treatment resistance can be elucidated (e.g. EGF-receptor mutations causing resistance to anti-cancer drugs).

Liver biopsy is used for example to diagnose the cause of chronic liver disease that results in an enlarged liver or abnormal liver test results caused by elevated liver enzyme activities (Rocken et al., 2001, Liver 21(6), 391-396).

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

Furthermore, all methods of the invention contain the step of contacting the cell preparation or cell lysate under essentially physiological conditions with at least one broad spectrum enzyme ligand immobilized on a solid support under conditions allowing the binding of the enzyme to said broad spectrum enzyme ligand.

The contacting under essentially physiological conditions has the advantage that the interactions between the ligand, the cell preparation (i. e. the enzyme to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

Preferably, "essentially physiological conditions" may comprise 50-200 mM NaCl or KCl, pH 6.5-8.5, 20-45°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2mM, wherein more preferably the buffer is selected from the group consisting ofTris-HCl or HEPES.

In the context of the present invention, the term "under conditions allowing the binding of the enzyme to said broad spectrum enzyme ligand" includes all conditions under which such binding is possible. This includes the possibility of having the solid support on an immobilized phase and pouring the lysate onto it. In another preferred embodiment, it is also included that the solid support is in a particulate form and mixed with the cell lysate.

In a preferred embodiment, the binding between ligand and enzyme is a non covalent, reversible binding, e.g. via salt bridges, hydrogen bonds, hydrophobic interactions or a combination thereof.

Additionally, the methods of the invention include the step of eluting the enzyme or enzymes from the ligand immobilized on the solid support.

Such methods are principally known in the art and depend on the nature of the ligand enzyme interaction. Principally, change of ionic strength, the pH value, the temperature or incubation with detergents are suitable methods to dissociate the target enzymes from the immobilized ligand. The application of an elution buffer can dissociate binding partners by extremes of pH value (high or low pH; e.g. lowering pH by using 0.1 M citrate, pH2-3), change of ionic strength (e.g. high salt concentration using Nal, KI, MgCl2, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinity chromatography. Mol. Biotechnol. 20(1), 41-47).

With these rather non-specific methods most or all bound proteins will be released and then need to be analysed by mass spectrometry (or alternatively by detection with antibodies, see below).

The method according to the 4^{th} aspect of the present invention further includes the step of contacting the bound enzymes with a compound to release at least one bound enzyme. This contacting preferably also occurs under essentially physiological conditions.

One advantage of using a compound of interest for elution instead of the non-specific reagents described above is that not all bound proteins are released but only a subfraction, preferably the enzyme class of interest. Consequently fewer proteins need to be identified by mass spectrometry resulting in faster analysis and more analytical depth (sensitivity) for the enzyme class of interest.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

After the elution or contacting, in some cases the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

According to the present invention, the eluted enzyme or enzymes or coeluted binding partners (see below) as well as the released enzymes according to the method of the fourth aspect of the invention are preferably characterized by mass spectrometry. Alternatively, it is throughout the invention also possible to perform this characterization with specific antibodies directed against the respective enzyme or coeluted binding partner.

The identification of proteins with mass spectrometric analysis (mass spectrometry) is known in the art (Shevchenko et al., 1996, Analytical Chemistry 68: 850-858, (Mann et al., 2001, Analysis of proteins and proteomes by mass spectrometry, Annual Review of Biochemistry 70, 437-473) and is further illustrated in the example section.

As an alternative to mass spectrometry analysis, the eluted enzyme or enzymes (including coeluted binding partners, for example enzyme subunits or scaffold proteins), can be detected by using specific antibodies directed against a protein of interest.

Furthermore, in another preferred embodiment, once the identity of the eluted enzyme or enzymes has been established by mass spectrometry analysis, each enzyme of interest can be detected with specific antibodies directed against this enzyme.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

Multiple assays can be performed using several antibodies in parallel, for example directed against many members of an enzyme family (Pelch et al., Kinetworks Protein Kinase Multiblot analysis. Chapter 8, pages 99-112 in: Cancer Cell Signalling. Methods and Protocols. Editor: David M. Terrian. Humana Press, Totowa, USA, 2002).

These assays can not only be configured in a way to detect and quantify an enzyme of interest, but also to analyse posttranslational modification patterns such as phosphorylation. For example, the activation state of a kinase can be determined by probing its phosphorylation status with specific anti-phosphotyrosine, anti-phosphoserine or anti-phosphothreonine antibodies. It is known in the art how to select and use such anti-phospho antibodies (Zhang et al., 2002. Journal of Biological Chemistry 277, 43648-43658).

According to a preferred embodiment of the method of the invention according to the 2^{nd} aspect, by characterizing the enzyme, it is determined whether the administration of the compound results in a differential expression or activation state of the enzyme. Therefore, by administration of the compound, either the expression of the enzyme may be changed or the activation state of the enzyme may be changed.

In this context, a change in the expression of the enzyme may preferably either mean that more or that less enzyme is produced in the cell.

By change of the activation state, it is preferably meant that either the enzyme is more active after administration of the compound or less active after the administration of the compound. It can also mean that the affinity of the enzyme for the immobilized ligand is increased or decreased (e.g. change of the activation state of a kinase through phosphorylation by an upstream kinase; or binding of the compound to an allosteric regulatory side of the enzyme and thereby altering the conformation of the ATP-binding pocket of an ATP-binding enzyme).

According to a preferred embodiment of the method of the invention according to the 1^{st} aspect, the protein preparation is incubated, preferably under essentially physiological conditions, with a compound as defined below. In consequence, only enzymes not binding to the compound are subsequently bound to the ligand, eluted and characterized.

According to a preferred embodiment of the method according to the 3^{rd} aspect of the invention, in step c) the aliquot is contacted, preferably under essentially physiological conditions, with the compound before the incubation with the ligand. In consequence, only enzymes not binding to the compound are subsequently bound to the ligand, eluted and characterized.

In a preferred embodiment of the method of the invention according to the third aspect, a reduced detection of the enzyme in the aliquot incubated with the compound indicates that the enzyme is a direct target of the compound. This results from the fact that in step c) of this method of the invention, the compound competes with the ligand for the binding of the enzyme. If less enzyme can be detected in the aliquot incubated with the compound, this means preferably that the compound has competed with the inhibitor for the interaction with the enzyme and is, therefore, a direct target of the enzyme and vice versa.

According to a preferred embodiment of the method of the invention according to the fourth aspect, this method is performed as a medium or high throughput screening. Such assays are known to the person skilled in the art (Mallari et al., 2003, A generic high-throughput screeing assay for kinases: protein kinase A as an example, Journal of Biomolecular Screeing 8, 198-204; Rodems et al., 2002, A FRET-based assay platform for ultra-high density screening of protein kinases and phosphatases, Assay and Drug Development Technologies 1 (1PT1), 9-19).

Essential to the methods according to the second, third and fourth aspect of the invention is the provision of a compound which is supposed to interact with the enzyme. Principally, according to the present invention, such a compound can be every molecule which is able to interact with the enzymes. Preferably, the compound has an effect on the enzyme, e. g. a stimulatory or inhibitory effect.

Preferably, said compound is selected from the group consisting of synthetic or naturally occurring chemical compounds or organic synthetic drugs, more preferably small molecules, organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 5000 Da, more preferred less than 2000 Da, even more preferred less than 1000 Da and most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr Med Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfil the specific requirements of the individual binding pocket within a domain family it may be necessary to optimise the natural product structure by chemical variation. Solid-phase chemistry is said to become an efficient tool for this optimisation process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

The test compounds that can elute target enzymes from the immobilized ligands (4^{th} aspect of the invention) may be tested in conventional enzyme assays. In the following, exemplary assays will be described that can be used to further characterize these compounds. It is not intended that the description of these assays limits the scope of the present invention.

### Protease assay

An exemplary protease assay can be carried out by contacting a protease with a double labeled peptide substrate with fluor (e.g. EDANS) and quencher chromophores (e.g. DABCYL) under appropriate conditions and detecting the increase of the fluorescence after cleavage.

The substrate contains a fluorescent donor near one end of the peptide and an acceptor group near the other end. The fluorescence of this type of substrate is initially quenched through intramolecular fluorescence resonance energy transfer (FRET) between the donor and acceptor. When the protease cleaves the substrates the products are released from quenching and the fluorescence of the donor becomes apparent. The increase of the fluorescence signal is directly proportional to the amount of substrate hydrolysed (Taliani, M. et al, 1996, Methods 240: 60-7).

### Phosphodiesterase assay

A cell lysate of human leukocytes (U937) cells may be prepared in a suitable buffer and serves as source of the PDE enzyme. After 20 minutes of incubation at 25°C with [3H]cAMP as substrate in incubation buffer (50 mM Tris-HCl, ph 7.5, 5 mM MgCl2) the [3H]Adenosine is quantified (Cortijo et al., 1993, British Journal of Pharmacology 108, 562-568).

### In vitro enzyme activity assay for protein kinases

Briefly, a fluorescein-labeled peptide substrate may be incubated with the tyrosine kinase (e.g. Lck), ATP and an anti-phosphotyrosine antibody. As the reaction proceeds, the phosphorylated peptide binds to the anti-phosphotyrosine antibody, resulting in an increase in the polarization signal. Compounds that inhibit the kinase result in a low polarization signal.

Alternatively, the assay can be configured in a modified indirect format. A fluorescent phosphopeptide is used as a tracer for complex formation with the anti-phospho-tyrosine antibody yielding a high polarization signal. When unlabeled substrate is phosphorylated by the kinase, the product competes with the fluorescent phosphorylated peptide for the antibody. The fluorescent peptide is then released from the antibody into solution resulting in a loss of polarization signal. Both the direct and indirect assays can be used to identify inhibitors of protein tyrosine kinase activity (Seethala, 2000, Methods 22, 61-70; Seethala and Menzel, 1997, Anal. Biochem. 253, 210-218;Seethala and Menzel, 1998, Anal. Biochem. 255, 257-262).

This fluorescence polarization assay can be adapted for the use with protein serine/threonine kinases by replacing the antiphophotyrosine antibody with an anti-phosphoserine or anti-phosphothreonine antibody (Turek et al., 2001, Anal. Biochem. 299, 45-53, PMID 11726183; Wu et al., 2000, J. Biomol. Screen. 5, 23-30, PMID 10841597).

The compounds identified in the method according to the 4^{th} aspect of the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

Preferably, lead optimisation is supported with a method according to the 2^{nd}, 3^{rd} and 4^{th} aspect of the present invention, more preferably with a method according to the 4^{th} aspect. The results of these methods may provide guidance to medicinal chemists or to another person skilled in the art how to further optimize compounds with respect to e.g. selectivity. One use of such a library is finally described in, for example, Wakeling AE, Barker AJ, Davies DH, Brown DS, Green LR, Cartlidge SA, Woodburn JR. Specific inhibition of epidermal growth factor receptor tyrosine kinase by 4-anilinoquinazolines. Breast Cancer Res Treat. 1996;38(1):67-73.

The enzyme which may be characterized according to the present invention, is preferably selected from the group consisting of a kinase, a phosphatase, a protease, a phosphodiesterase, a hydrogenase, a dehydrogenase, a ligase, an isomerase, a transferase, an acetylase, a deacetylase, a GTPase, a polymerase, a nuclease and a helicase.

Preferably, the protein is a kinase, and more preferably a protein kinase. Equally preferred, the protein is a lipid kinase.

As already indicated above, it is essential to the present invention that the ligand is a broad spectrum ligand which is able to bind various, but not all enzymes of a given class of enzymes. Preferably, the ligand binds to 10 to 50 %, more preferably to 30 to 50 % of the enzymes of a given class of enzymes.

Preferably, the ligand is an inhibitor of the enzyme.

In a more preferred embodiment, the enzyme is a kinase and the ligand is a kinase inhibitor.

Preferably, this kinase inhibitor is selected from the group consisting of Bisindolylmaleimide VIII, Purvalanol B, CZC00007324 (linkable PD173955), CZC00008004.

Further ligands include indol ligand 91, quinazoline ligand 32 and a modified Staurosporine (see Example 5 to 7).

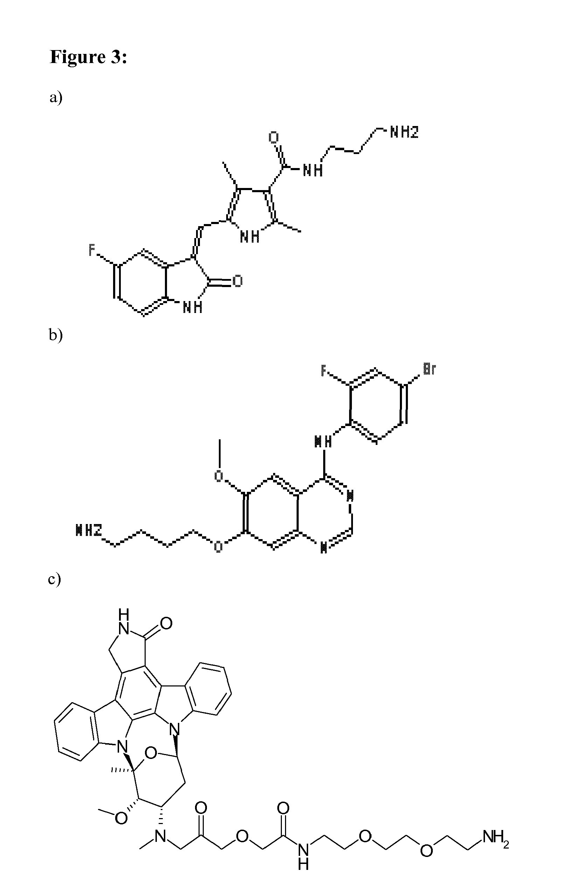

The structure of indol ligand 91 (5-[5-Fluoro-2-oxo-1,2-dihydro-indol-(3Z)-ylidenemethyl]-2,4-dimethyl-1H-pyrrole-3-carboxylic acid (3-amino-propyl)-amide) is given in Fig. 3. This compound is a molecule structurally similar to the kinase inhibitor Sutent (SU11248; Sun et al., 2003. J. Med. Chem. 46, 1116-1119). Indol ligand 91 can be covalently coupled to a suitable solid support material via the primary amino group and be used for the isolation of binding proteins. The synthesis of indol ligand 91 is described in Example 1. According to the invention, the expression "indol ligand 91" also includes compounds comprising the identical core but which have another linker, preferably coupled to the NH group not being part of the cyclic structures, for linkage to the solid support. Typically linkers have backbone of 8, 9 or 10 atoms. The linkers contain either a carboxy- or amino-active group.

According to a further preferred embodiment, the characterization of the enzyme is performed by characterizing co-eluted binding partners of the enzyme, enzyme subunits or post-translational modifications of the enzyme.

The basis of this preferred embodiment is that due to the use of essentially physiological conditions during the binding between the ligand and the enzyme, it is preferably possible to preserve the natural condition of the enzyme which includes the existence of binding partners, enzyme subunits or post-translational modifications. With the help of mass spectrometry (MS), it is possible not only to identify the enzyme, but also the co-eluted binding partners, enzyme subunits or said post-translational modifications.

According to a further preferred embodiment of the present invention, the characterization by mass spectrometry (MS) is performed by the identification of proteotypic peptides of the enzyme or of the binding partner of the enzyme. The concept of proteotypic peptides is described in detail in the example section. The idea is that the eluted enzyme or binding partner is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained for the enzyme or the binding partner with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample.

Preferably, the mass spectrometry analysis is performed in a quantitative manner, for example by using iTRAQ technology (isobaric tags for relative and absolute quantification) or cICAT (cleavable isotope-coded affinity tags) (Wu et al., 2006. J. Proteome Res. 5, 651-658).

According to a further preferred embodiment, the solid support is selected from the group consisting of agarose, modified agarose, sepharose beads (e. g. NHS-activated sepharose), latex, cellulose, and ferri- or ferromagnetic particles.

The broad spectrum enzyme ligand may be coupled to the solid support either covalently or non-covalently. Non-covalent binding includes binding via biotin affinity ligands binding to steptavidin matrices.

Preferably, the broad spectrum ligand is covalently coupled to the solid support.

Before the coupling, the matrixes can contain active groups such as NHS, Carbodimide etc. to enable the coupling reaction with compounds. The compounds can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the compound and non-covalent binding of biotin to streptavidin which is bound to solid support directly).

The linkage to the solid support material may involve cleavable and non-cleavable linkers. The cleavage may be achieved by enzymatic cleavage or treatment with suitable chemical methods.

Preferred binding interfaces for binding the compound of interest to solid support material are linkers with a C-atom backbone. Typically linkers have backbone of 8, 9 or 10 atoms. The linkers contain either, depending on the compound to be coupled, a carboxy- or amino-active group.

More complete coverage of an enzyme class can be achieved by using combinations of broad spectrum ligands.

Preferably, 1 to 10 more preferred 1 to 6, even more preferred 1 to 4 different ligands are used. Most preferred, 3 or 4 different ligands are used

In case that more than one ligand is used, each ligand is preferably on a different support.

However, it is equally preferred that when more than one ligand is used, at least two or all different ligands are present on one solid support.

In case that more than one ligand is used, it is preferred that the spectrum which each individual ligand can bind is different so that maximum coverage of the enzyme class can be achieved.

Preferably, each ligand binds to 10 to 50 %, more preferably to 30 to 50 % of the enzymes of a given class of enzymes.

According to a further preferred embodiment, by characterizing the enzyme or the compound enzyme complex, the identity of all or several of the members of an enzyme class in the cell is determined. This is due to the fact that by incubating the ligand with the cell lysate, potentially all enzymes being capable of binding to the ligand are isolated and later on characterized. Depending on the expression profile of the enzymes, the ligand is able to bind to all or some of the members of an enzyme class, which can thus be identified. In the case of kinases, the methods of the present invention enable the skilled person to identify and characterize the kinome expressed in a given cell.

Throughout the invention, it is preferred that the compound is different from the ligand, although identity is not excluded.

The invention further relates to a method for the production of a pharmaceutical composition, comprising the steps of:
a) identifying an enzyme compound complex according to the method of the fourth aspect of the present invention, and
b) formulating the compound to a pharmaceutical composition.

Therefore, the invention provides a method for the preparation of pharmaceutical compositions, which may be administered to a subject in an effective amount. In a preferred aspect, the therapeutic is substantially purified. The subject to be treated is preferably an animal including, but not limited to animals such as cows, pigs, horses, chickens, cats, dogs, etc., and is preferably a mammal, and most preferably human. In a specific embodiment, a non-human mammal is the subject.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the therapeutic can be delivered via a controlled release system. In one embodiment, a pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a preferred embodiment, the method further comprises the step of modulating the binding affinity of the compound to the enzyme. This can be accomplished by methods known to the person skilled in the art, e. g. by a chemical modification of various residues of the compound and subsequent analysis of the binding affinity of the compound to the enzyme.

The invention further relates to the use of at least one broad spectrum enzyme ligand immobilized on a solid support for the characterization of at least one enzyme or of an enzyme-compound complex. With respect to this use of the invention, all embodiments as described above for the methods of the invention also apply.

The invention is further illustrated by the following figures and examples, which are not considered as being limiting for the scope of protection conferred by the claims of the present application.

### Short description of the figures

- **Figure 1:**: Structures of kinobead ligands. The source and synthetic routes of the ligands are described in Example 1.
- Fig. 1a:: Kinobead ligand 1 (Bisindolylmaleimide VIII)
- Fig. 1b:: Kinobead ligand 2 (Purvalanol B)
- Fig. 1c:: Kinobead ligand 3 (CZC00007324, (7-(4-Aminomethyl-phenylamino)- 3-(2,6-dichloro-phenyl)-1-methyl-1H-[1,6]naphthyridin-2-one))
- Fig. 1d:: Kinobead ligand 4 (CZC00008004, 2-(4'-aminomethyl phenylamine)-5-fluoro-pyrimidin-4-yl)-phenyl-amine)
- **Figure 2:**: Signalokinome (see Example 2).
This figure shows a comparison of Drug pulldowns using kinobeads (left circle) and conventional imunoprecipitations with anti-phosphotyrosine antibody beads (right circle). In the Kinobeads experiment a total of 626 proteins were identified, of these are 100 kinases. In the immunoprecipitation (IP) experiment a total of 503 proteins were identified, 12 of these were kinases. Kinases identified with both experiments (common kinases) are listed in the overlap area. The result shows that with the kinobeads significantly more kinases were identified (100 kinases) compared to the anti-phosphotyrosine antibody beads (12 kinases).
- **Figure 3:**: Structures of kinobeads ligands 5, 6 and 7. The sythetic routes of the ligands are described in Example 5, 6 and 7.
- Fig. 3a:: Structure of kinobead ligand 5 (indol ligand 91)
- Fig. 3b:: Structure kinobead ligand 6 (quinazoline ligand 32)
- Fig. 3c:: Structure kinobead ligand 7 (modified Staurosporine)

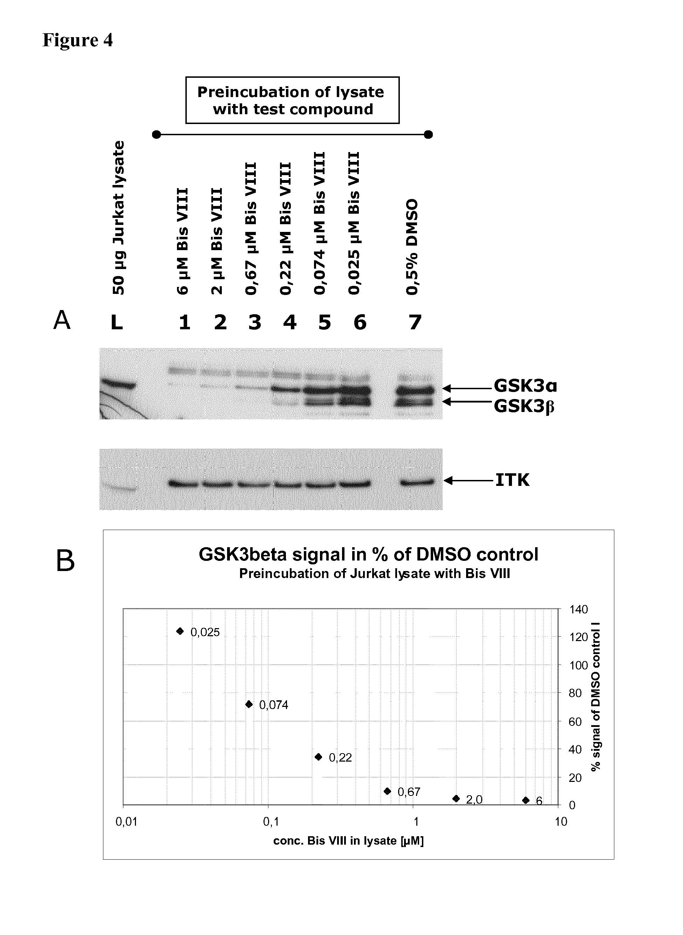

- **Figure 4:**: Quantitative protein affinity profile (PAP).
The figure shows in lysate competition with test compound Bis VIII and detection of proteins with Western blot analysis. The pulldown experiment was performed as described in Example 8 with Jurkat cell lysate samples containing 10 mg of protein. Input lysate (lane L; 50 µg protein) and SDS-eluates from kinobeads (lanes 1 to 7) were separated on a SDS-polyacrylamide gel, transferred to a membrane and probed with antibodies.
Fig. 4A: The Western blot was probed first with an antibody directed against GSK3beta. Secondary detection antibodies labeled with peroxidase were used for chemiluminescent detection. As a loading control a blot was probed with an anti-ITK antibody. Lane L: 50 µg of Jurkat lysate; lane 1: 6.0 µM Bis VIII; lane 2: 2.0 µM Bis VIII; lane 3: 0.67 µM Bis VIII; lane 4: 0.22 µM Bis VIII; lane 5: 0.074 µM Bis VIII; lane 6: 0.025 µM Bis VIII; lane 7: 0.5% DMSO (solvent control).

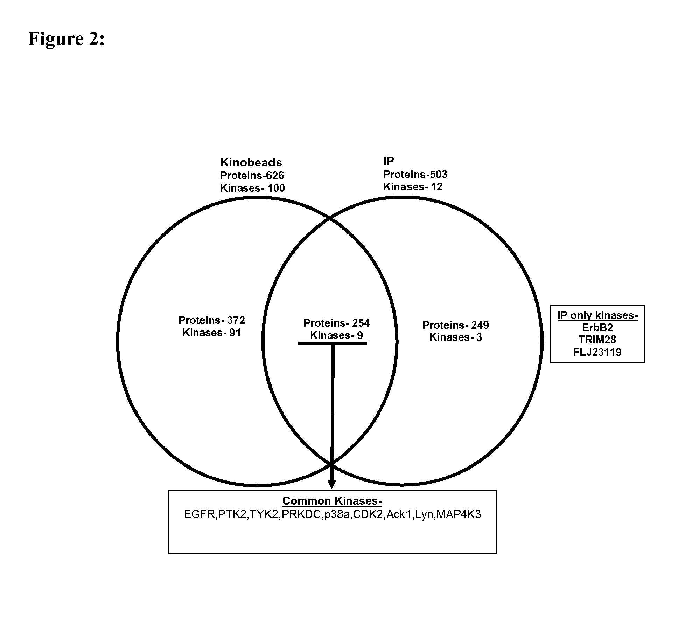

Fig. 4B: Concentration dependent competition of GSK3 beta binding to kinobeads by Bis VIII. The GSK3beta bands on the Western blot shown in Figure 2A were quantified and plotted against the concentration of Bis VIII added to the lysate.

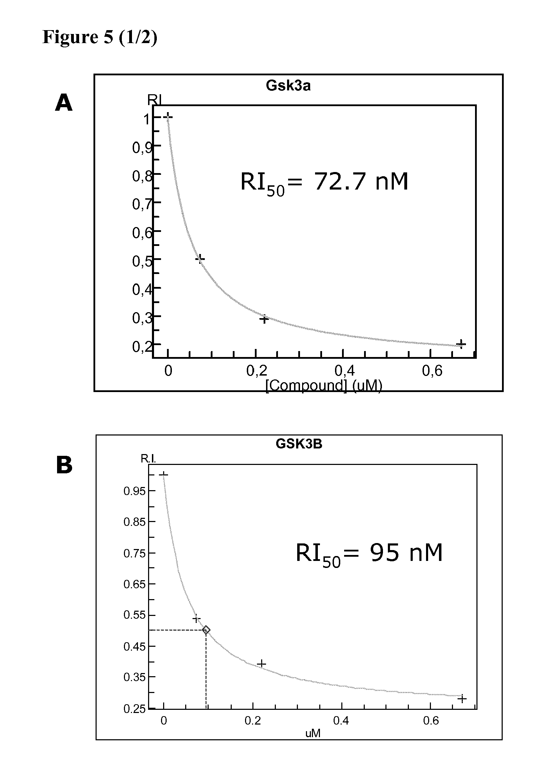

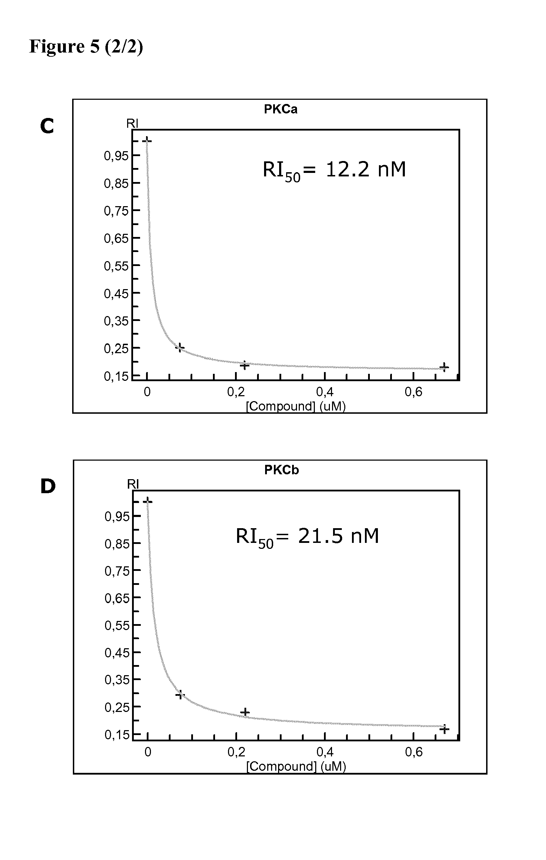

- **Figure 5:**: Quantitative protein affinity profile for kinases The results of the quantitative affinity profile experiment of example 8 are displayed for four kinases. Relative Intensity (RI) values are plotted against compound concentration (Bis VIII). The RI50 value represents the compound concentration at which the relative intensity of the MS signal for a given kinase is 50% compared to the DMSO control.
Fig. 5A: Curve for Glycogen Synthase Kinase 3 alpha (GSKa; RI50 =72.7 nM)
Fig. 5A: Curve for Glycogen Synthase Kinase 3 beta (GSKb; RI50 =95 nM)
Fig. 5C: Curve for protein kinase C alpha (PKCa; RI50 =12.2 nM)
Fig. 5D: Curve for protein kinase C beta (PKCb; RI50 =21.5 nM)

### Examples

### Example 1: Preparation of kinobeads

This example illustrates the preparation of kinobeads with 4 different ligands. These kinobeads were later used in example 2 and example 3.

Broad spectrum capturing ligands were covalently immobilized on a solid support through covalent linkage using suitable functional groups (e.g. amino or carboxyl or groups). Compounds that do not contain a suitable functional group were modified in order to introduce such a group. The necessary chemical methods are known in the medicinal chemistry literature and illustrated below.

### 1. Selection and synthesis of ligands

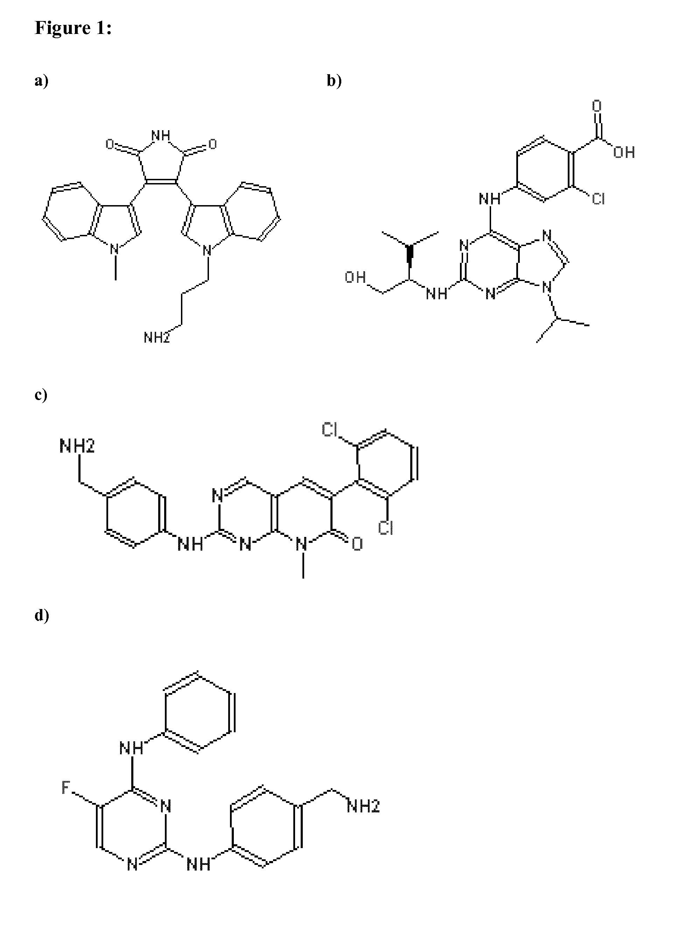

The following four broad specificity ligands (Kinobead ligands 1 to 4; Figure 1) were covalently coupled to beads in separate reactions as described below and then the four types of beads were mixed and used for the drug pulldown experiments.

**Kinobead ligand 1:** Bisindolylmaleimide VIII-Acetate (Chemical Formula: C₂₄H₂₂N₄O₂. CH₃COOH; MW 398.5; CAS number 138516-31-1; Alexis Biochemicals, AXXORA Deutschland GmbH, Crünberg; Cat- ALX-270-056).

**Kinobead ligand 2:** Purvalanol B (Chemical composition: C₂₀H₂₅CN₆O₃; MW 441.92; CAS number 212844-54-7; Tocris Biochemicals Cat- 1581, BIOTREND Chemikalien GmbH Köln, Germany).

**Kinobead ligand 3:** CZC00007324; (7-(4-Aminomethyl-phenylamino)-3-(2,6-dichlorophenyl)-1-methyl-1H-[1,6]naphthyridin-2-one).

### Synthesis of kinobead ligand 1: 7-(4-Aminomethyl-phenylamino)-3-(2,6-dichloro-phenyl)-1-methyl-1H-[1,6]naphthyridin-2-one

The first seven steps of the synthesis of CZC00007324 were performed as described in Klutchko, S.R. et al., 1998, Journal of Medicinal Chemistry 41, 3276-3292. The remaining steps were performed as described below.

**Steps 1-7:** 6-(2,6-Dichlorophenyl)-2-methanesulfonyl-8-methyl-8*H*-pyrido[2,3-*d*]pyrimidin-7-one was synthesized from 4-chloro-2-methylsulfanyl-5-pyrimidinecarboxylate ethyl ester following the procedure in J. Med. Chem. 1998, 41, 3276-3292.

### Step 8: {4-[3-(2,6-Dichloro-phenyl)-1-methyl-2-oxo-1,2-dihydro-[1,6]naphthyridin-7 ylamino] benzyl}-carbamic acid tert-butyl ester

6-(2,6-Dichlorophenyl)-2-methanesulfonyl-8-methyl-8*H*-pyrido[2,3-*d*]pyrimidin-7-one (0.100g, 0.2mmol) and 3-(*N*-Boc-methylamino)aniline (0.421g, 2.0mmol) were mixed as solids and heated to 140°C for 30 mins. The crude reaction mixture was dissolved in dichloromethane and washed with 2N HCl (aq) x 2. The organic layer was dried with anhydrous magnesium sulfate, filtered and concentrated. The crude product was recrystallised from hot ethyl acetate to afford {4-[3-(2,6-Dichloro-phenyl)-1-methyl-2-oxo-1,2-dihydro-[1,6]naphthyridin-7-ylamino] benzyl}-carbamic acid *tert*-butyl ester as a yellow solid (0.031g- 25%). ¹H NMR (DMSO-*d*₆) δ 10.18 (s, 1H); 8.83 (s, 1H); 7.76 (d, 2H); 7.58 (d, 2H); 7.46 (dd, 1H); 7.32 (brt, 1H); 7.23 (d, 2H); 4.10 (d, 2H); 3.66 (s, 3H); 1.40 (s, 9H). LCMS: method A, RT=5.60min.

### Step 9: 7-(4-Aminomethyl-phenylamino)-3-(2,6-dichloro-phenyl)-1-methyl-1H-[1,6]naphthyridin-2-one

{4-[3-(2,6-Dichloro-phenyl)-1-methyl-2-oxo-1,2-dihydro-[1,6]naphthyridin-7-ylamino] benzyl}-carbamic acid *tert*-butyl ester (0.026g, 0.05mmol) was dissolved in methanol (3ml) and hydrochloric acid (4N in dioxane, 1.2ml) was added. The reaction was stirred at room temperature for 1.5 hours when HPLC showed no remaining starting material. The solvent was removed *in vacuo.* The residue was dissolved in water and the solution basified with sodium carbonate (sat., aq.). The resulting precipitate was collected and dried to afford 7-(4-Aminomethyl-phenylamino)-3-(2,6-dichloro-phenyl)-1-methyl-1H-[1,6]naphthyridin-2-one (0.021g -100%) as a yellow solid. ¹H NMR (DMSO-*d*₆) □ δ □ 10.20 (brd, 1H); 8.83 (d, 1H); 7.90 (d, 1H); 7.76 (d, 1H); 7.72 (d, 1H); 7.60 (dd, 2H); 7.47 (ddd, 1H); 7.33 (d, 1H); 7.24 (d, 1H); 4.07 (d, 2H); 3.66 (s, 3H). LCMS: method A, RT=4.44min, [MH⁺=426].

**Kinobead ligand 4:** CZC00008004. 2-(4'-aminomethyl phenylamine)-5-fluoro-pyrimidin-4-yl)-phenyl-amine. Chemical formula C₁₇H₁₆N₅F. MW 309.34. This is an analog of CZC00004919.

### Synthesis of CZC00008004 - (2-(4'-aminomethyl phenylamine)-5-fluoro-pyrimidin-4-yl)-phenyl-amine

### Step 1: 2,4-Dichloro-5-fluoro-pyrimidine

Phosphorus oxychloride (2ml) was added to 5-fluorouracil (1g, 7.688mmol) followed by phosphorus pentachloride (3.28g, 15.76mmol), the mixture was heated and stirred at 110°C for 5 hours and then allowed to cool. The excess phosphorus oxychloride was slowly hydrolised in a bath of ice / water mixture (10ml). The aqueous mixture was extracted with diethyl ether (3 x 10ml). The organic layers were combined, washed with saturated sodium bicarbonate (10ml) followed by saturated sodium chloride (10ml), dried with anhydrous magnesium sulfate and then filtered. The solvent was removed by evaporation at 350mmHg to leave a viscous oil which slowly crystallised to afford the title compound (1.22g - 95%). The compound was used without further analysis on the next step.

### Step 2: (2-Chloro-5-fluoro-pyrimidin-4-yl)-phenylamine

To a solution of 2, 4-Dichloro-5-fluoro-pyrimidine (0.550g, 3.30mmol) in dimethylformamide (13ml) was added aniline (0.301ml, 3.30mmol) and *N-*ethyldiisopropylamine (0.602ml, 3.63mmol) and the mixture stirred at room temperature for 18 hours. The mixture was quenched with ethyl acetate (20ml) and washed with saturated ammonium chloride (20ml) and the organic layer removed. The aqueous layer was washed with ethyl acetate (20ml) and the combined organic layers washed with water (20ml), saturated sodium chloride (10ml) and dried with anhydrous magnesium sulfate. The solvent was removed by evaporation and the resulting solid subjected to column chromatography (silica, ethyl acetate (0 to 20%)/ petrol ether) to afford the title compound (0.532g - 72%). LCMS: method A, RT=4.67min, [MH⁺=224].

### Step 3: [4-(5-Fluoro-4-phenylamino-pyrimidin-2-ylamino)-benzyl]-carbamicacid tert-butyl ester

(2-Chloro-5-fluoro-pyrimidin-4-yl)-phenylamine (0.087g, 0.39mmol) and 4[*N*-Boc aminomehtyl]aniline (0.087g, 0.39mmol) were mixed together. A stirrer bar was added and the flask placed in a oil bath at 110°C for 20 mins. The mixture was cooled, the residue dissolved in 3ml of Dichloromethane/Methanol (99:5) and loaded up on a Flash Chromatography cartridge and purified using ethyl acetate (20 to 60%) in petrol ether to give the desired compound as a yellow solid (0.051g - 32%). ¹H NMR (400 MHz, CDCl₃-*d*₆) δ 7.85 (d, 1H); 7.50 (dd, 2H); 7.39 (d, 2H); 7.27 (t, 2H); 7.12-7.00 (m, 3H); 6.81 (s, 1H); 6.66 (s, 1H); 4.67 (s, 1H), 4.17 (d, 2H), 1.36 (s, 9H). LCMS: method B, RT=9.20 min, [MH⁺=410].

### Step 4: (2-(4'-aminomethyl phenylamine)-5-fluoro-pyrimidin-4-yl)-phenyl-amine

To a solution of [4-(5-Fluoro-4-phenylamino-pyrimidin-2-ylamino)-benzyl]-carbamicacid *tert*-butyl ester (0.055g, 0.134mmol) in methanol (5ml) HCl (4N in dioxane) (2ml) and the reaction was stirred at room temperature for 1 hour. The solvent was removed by evaporation. Water (5ml) was added and the pH of the solution was raised to 8 by addition of sodium bicarbonate. The resulting precipitate was filtered and dried to afford the title compound (0.035g - 84%). ¹H NMR (400 MHz, DMSO-*d*₆) δ 7.90 (d, 1H); 7.70 (dd, 2H); 7.55 (dd, 2H); 7.35 (t, 2H); 7.20 (d, 2H); 7.10 (t, 1H); 3.80 (s, 2H). LCMS: method B, RT=5.022min, [MH⁺=310].

All reactions were carried out under inert atmosphere. NMR spectra were obtained on a Bruker dpx400. LCMS was carried out on an Agilent 1100 using a zorbax SBC-18, 4.6mmx150mm-5µ column. Column flow was 1ml/min and solvents used were water and acetonitrile (0.1%TFA) with an injection volume of 10ul. Wavelengths were 254 and 210nm. Methods are described below.

**Table 1: Analytical methods**

| **Method** | **Easy Access Method Name** | **ChemStation Method Name** | **Flow Rate** | **Solvent** | **Run Time** |
|---|---|---|---|---|---|
| A | Analytical positive 7mn | ANL_POS7.M | 1ml/min | 0-2.5min 5-95% MeCN | 7 min |
| | | | | 2.5-6min | |
| | | | | 95% MeCN | |
| B | Analytical positive Ion | ANAL_POS.M | 1ml/min | 0-11min 5-95% MeCN | 15 min |
| | | | | 11-13min 95% MeCN | |

**Table 2: Abbreviations used in chemistry protocols**

| | |
|---|---|
| aq | aqueous |
| d | doublet |
| DMSO | dimethyl sulfoxide |
| g | gram |
| HCl | Hydrochloric acid |
| HPLC | high pressure liquid chromatography |
| LCMS | liquid chromatography - mass spectrometry |
| m | multiplet |
| mins | minute |
| mmol | millimole |
| N | Normal |
| NMR | nuclear magnetic resonance |
| q | quartet |
| RT | retention time |
| S | singlet |
| sat | saturated |
| t | triplet |

### 2. Immobilization of ligands containing amine groups

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (SIGMA, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at roomtemperature on the end-over-end shaker over night. Washed beads were stored in isopropanol or immediately used for binding reactions.

### 3. Immobilization of ligands containing carboxyl groups

The compounds were coupled under basic condition to reversed NHS-Sepharose beads (PyBroP chemistry) as outlined below.

### Washing of beads

Step 1: Use 1 ml (settled volume) NHS-sepharose/beads for a standard coupling reaction (NHS-activated Sepharose 4 Fast Flow provided in isopropanol, Amersham Biosciences, 17-0906-01).
Step 2: Wash the beads 3 times with 10 ml DMSO and once with 10 ml anhydrous DMSO (Dimethylsulfoxid, Fluka, 41648, H₂0 <= 0.005%); centrifugation steps: 1 min, 1.200 rpm, room temperature; discard supernatant into non-halogenous solvent waste.
Step 3: After last washing step resuspend beads in one volume of anhydrous DMSO.

### Reversing the NHS beads

This step is designed for 1 mL beads, adjust accordingly for any other bead volumes. NHS beads have a capacity of 20 µmol/mL. Therefore, 20% reversed beads should have a capacity of 4 µmol/mL.

Make a 4:1 ratio mixture of Aminoethanol and Ethylenediamine, then add Triethtylamine to the mixture.

(200 µmol total) (10 x capacity of 1 mL NHS beads)
1: 9.66 µL 16.56 M Aminoethanol (2-Aminoethanol, Aldrich, 11.016-7) (total 160 µmol)
2: 2.68 µL 14.92M Ethylenediamine (Fluka, 03350) (total 40 µmol).
3: 15 µL 7.2 M Triethylamine (TEA) (SIGMA, T-0886, 99% pure) Mixture will be split in two phases, but that is OK.

Add mixture to washed/resuspended NHS beads and incubate 16 hours (over night) at room temperature on the end-over-end shaker.

**PyProB coupling.** This procedure does not require a preactivation step, activation and coupling occurs in-situ.
1. Dissolve PyBroP (Bromo-tris-pyrrolidino-phosphonium hexafluorophosphate; supplier: NOVABIOCHEM) in waterfree Dimethylformamide (DMF) to a final concentration of 100 mM (46.62 mg/mL), the solution can be used for up to one day.
2. Dissolve 35 µL of Diisopropylethylamine (DIEA) in 1 mL waterfree DMF, final concentration 200 mM.
3. Wash 1 mL of reversed beads 3 times with 15 mL DMSO. Centrifugation step: 1 minute at 1,200 rpm room temperature. Discard supernatant.
4. Wash reversed beads 3 times with 15mL water-free DMF. Centrifugation step 1 minute, 1,200 rpm room temperature. Discard supernatant.
5. Suspend reversed sepharose beads in 1 mL of waterfree DMF.
6. Add 100 µL of Diisopropylethylamine (DIEA) solution.
7. Add compound (1 µmol/mL reversed Sepharose beads; corresponds to 10 µL 100 mM compound / mL reversed sepharose beads) from DMF-solution in the required amount to the beads.
8. Mix until a homogenous suspension is obtained.
9. Centrifuge 1 minute at 1200 rpm at room temperature, remove 20 µL supernatant and dilute in 30 µL methanol (MeOH) for the starting value for HPLC analysis.
10. Add 100 µL of PyBroP solution to the suspension and mix again.
11. Incubate over night on the end-over-end mixer at room temperature.
12. Centrifuge 1 minute at 1200 rpm at room temperature, remove 20 µL supernatant and dilute in 30 µL methanol (MeOH) for the coupled value for HPLC analysis.

### Blocking of the beads

1. Block the beads by adding 100 µL 100 mM NHS-Acetate (see below how to make blocking reagent).
2. Incubate over-night at room temperature on the end-over-end shaker.

Blocking reagent (NHS activated acetic acid):
1. Prepare 200 mM solutions of DCCD and NHS in acetonitrile (∼ 5 mL of each).
2. Mix equal volumes of the 200 mM NHS and DCCD in a 20 mL clear glass vial.
3. Per 1 mL total volume NHS/DCCD mix, add 11.4 µL 17.49 M acetic acid (2x molar excess) (Merck, 1.00063.1000).
4. Mix thoroughly. A precipitate will form after about 2 minutes (crystals of the urea derivative).
5. Allow the reaction to sit at room temperature at least overnight before further use.

### Washing of beads

1. Wash the beads 2 times with 14 ml DMSO (e.g. FLUKA, 34869 or equivalent), then with 2 x 14 mL isopropanol (Merck, 1.00983.1000, pro analysis). Centrifuge steps: 1 minute at 1200 rpm at room temperature. Remove supernatant between washes.
2. Resuspend the beads with 1 mL isopropanol to make a 50% slurry for storage at -20°C or use immediately for binding reactions with cell lysates.

**Table 3: Abbreviations used in coupling protocols**

| | |
|---|---|
| DCCD | Dicyclohexylcarbodiimide |
| DIEA | Disopropylethylamine |
| DMSO | Dimethyl sulfoxide |
| DMF | Dimethylformamide |
| NHS | N-hydroxysuccinimide |
| PyBroP | Bromo-tris-pyrrolidino-phosphonium hexafluorophosphate |
| TEA | Triethylamine |

### Example 2: Signalokinome

This example illustrates the treatment of cells with compounds (see particularly the second aspect of the invention). HeLa cells were treated with epidermal growth factor (EGF), a cell lysate was prepared and analysed using Kinobeads (experimental protocol in section 2.1) and mass spectrometry. The preparation of Kinobeads is described in Example 1.
In parallel the cell lysate was subjected to imunoprecipitation with an anti-phosphosphotyrosine antibody (experimental protocol in section 2.2) and analysed by mass spectrometry.
The result (Figure 2) shows that kinobeads identify significantly more kinases (Table 8) compared to the immunoprecipitation procedure (Table 6).

### 1. Preparation of the biological sample (cell lysate)

### 1.1 Cell culture and treatment of cells

**Cell culture.** HeLa cells (American Type Culture Collection-No CCL-2) were grown in MEM medium (without L-Arginine and without L-Glutamine; Promocell C-75280), 10% dialyzed Fetal Bovine Serum (Gibco, 26400-044), 1% 100x non-essential amino acids (Cambrex, BE13-114E), 1mM Sodium Pyruvate (Gibco, 11360-039), 2mM L-glutamine (Gibco, 25030-032), 40mg/L 12C or 13C L-Arginine (12C Arginine- Sigma, A6969) (13C Arginine- Cambridge Isotope Laboratories Inc., CLM-2265) at 37°C, 5% CO2.

**Cell propagation.** After cells had reached confluency in a 15cm dish, cells were split 1 to 10 for further growth. Cells were split by first removing the supernatant media, then briefly washing the cells with 15 mL PBS buffer (Gibco, 14190-094). After removal of the PBS, the cells were detached from the plate by adding 2mL trypsin-EDTA solution (Gibco, 25300-054) per 15cm plate and incubating the plate for 10 minutes at 37°C. After detachment of the cells, 8 mL MEM growth medium (see above) was added per 15cm plate. 1mL of this solution was put on fresh 15cm plates and 24 mL MEM media (see above) was added. Plates were again incubated at 37C 5% CO2 until the cells were confluent (∼3-4 days).

**EGF treatment of cells.** One day prior to treatment of the cells with Epidermal Growth Factor (EGF), the cell growth medium was removed by aspiration and 20 mL fresh MEM medium (see above) was added except that the medium was supplemented with 0.1 % Fetal Bovine Serum (FBS) instead of 10% FBS. The cells were incubated in this starvation medium overnight at 37°C, 5%CO2. After cell starvation, 3 µL 1 mg/mL recombinant human EGF (Biomol, 50349-1) was added to each 15cm plate (final EGF concentration = 150ng/mL medium). The plates were incubated at 37°C, 5%CO2 for 10minutes prior to harvesting.

**Cell harvesting.** Cells were harvested by pouring off of the EGF-containing medium, washing of each 15cm plate once with 10mL ice-cold PBS buffer, and scraping the plate with a rubber policeman in order to detach the cells. The cells were transferred into a 50mL Falcon tubes (Becton Dickinson, 352070) and centrifuged for 10 minutes at 1500 rpm in a Heraeus Multifuge 3SR. The supernatant was aspirated and the cell pellet was resuspended in 50 mL ice-cold PBS buffer. After centrifugation and aspiration of the supernatant cell pellets were quickly frozen in liquid nitrogen and then stored at -80°C.

### 1.2 Preparation of cell lysates

The HeLa cell lysate was prepared by mechanical disruption in lysis buffer solution under gentle conditions that maintain the structure and function of proteins.

The following steps were carried out:
- Thaw the tissue quickly at room temperature or 37°C, then transfer tissue to a glass bottle containing the 1x lysis buffer (use a vial big enough to be used with Polytron PT 3100 homogenizer)
- Lyse the organ/tissue with 4x 10 sec pulses at 5000-7000 rpm at 4°C in the cold room
- Transfer the homogenate into precooled 50 ml falcon tubes
- Incubate homogenate on ice for 30 min
- Spin cells for 10 min at 6000 g at 4°C (6.000 rpm in Sorvall SLA600, precooled)
- Transfer supernatant to a UZ-polycarbonate tube (Beckmann, 355654)
- Spin supernatant for 1 h at 145.000 g at 4°C (40.000 rpm in Ti50.2, precooled)
- Save supernatant (remove and discard most of the lipid layer if possible), transfer supernatant into a glass bottle and store on ice
- Determine protein concentration by Bradford assay (BioRad). Typical protein concentrations are in the range of 5-10 mg/ml.
- Prepare aliquots in 15 to 50 ml Falcon tubes
- Freeze aliquots in liquid nitrogen and store them at -80°C

### Preparation of 100 ml 1x lysis buffer with 0.8% NP40:

Combine the following solutions or reagents and add destilled water to a final volume of 100 ml: 20 ml 5x lysis buffer (see below), 100 µl 1M DTT, 5 ml 0.5 M NaF, 4 ml 20% NP40, 4 complete EDTA-free tablets (protease inhibitor cocktail, Roche Diagnostics, 1 873 580), add distilled water to 100 ml.

**Table 4: Preparation of 5x-lysis buffer**

| **Substance:** | **Stock solution** | **Final conc. in 1x lysis buffer** | **Add for 11 5 x lysis buffer** |
|---|---|---|---|
| **Tris/HCl pH 7.5** | 1 M | 50 mM | 250 ml |
| **Glycerol** | 87% | 5% | 288 ml |
| **MgCl₂** | 1M | 1.5 mM | 7.5 ml |
| **NaCl** | 5M | 150 mM | 150 ml |
| **Na₃VO₄** | 100 mM | 1 mM | 50 ml |

These solutions were obtained from the following suppliers:
1M Tris/HCl pH 7.5: Sigma, T-2663; 87% Glycerol: Merck, cat.no. 04091.2500; 1 M MgCl₂: Sigma, M-1028; 5 M NaCl: Sigma, S-5150.
The 5x concentrated lysis buffer was filtered through a 0.22 µm filter and stored in 40 ml aliquots at -80°C.

### Preparation of stock solutions used in this protocol:

### Preparation of 100 mM Na₃VO₄ stock solution:

Dissolve 9.2 g Na₃VO₄ in 400 ml distilled water.
1) Adjust the pH to 10.0 using either 1 N NaOH or 1 N HCl. The starting pH of the sodium orthovanadate solution may vary with batch. At pH 10.0 the solution will be yellow.
2) Boil the solution until it turns colorless (approximately 10 min).
3) Cool to room temperature.
4) Readjust the pH to 10.0 and repeat steps 2 and 3 until solution remains colorless and the pH stabilizes at 10.0.
5) Adjust the volume to 500 ml with distilled water.
6) Freeze aliquots at -20°C. Aliquots can be stored for several months.

### Preparation of 500 mM NaF stock solution:

Dissolve 21.0 g NaF (Sigma, S7920) in 500 ml distilled water. Filter solution through a 0.22 µm filter and store at 4°C.

### Preparation of 20% NP40-solution:

Weigh 40.0 g NP40 (Sigma, Igepal-CA630, catatogue No. 13021). Add distilled water up to 200 g. Mix completely and store solution at room temperature.

### Preparation of 1 M DTT solution:

Dissolve 7.7 g DTT (Biomol, catalogue No. 04010) in 50 ml distilled water. Filter solution through 0.22 µm filter and freeze 400 µl aliquots at -20°C. Aliquots can be stored for several months.

### 2. Binding reactions

### 2.1 Contacting of the "kinobeads" (immobilized capturing ligands) with the cell lysate

The kinobeads (immobilized capturing ligands) were contacted with cell lysate prepared from HeLa cells under conditions that allow the binding of the proteins in the lysate to the ligands. The binding conditions were close to physiological by choosing suitable buffer conditions preserving the function of the proteins. After removing non-captured proteins through a gentle washing procedure the bound proteins were contacted with a test compound which led to the elution of proteins.

### 2.1.1 Preparation of DP-buffers

**Table 5: Preparation of 5x-DP buffer**

| **Substance:** | **Stock solution** | **Final conc. in 1x lysis buffer** | **Add for 11 5 x lysis buffer** |
|---|---|---|---|
| **Tris/HCl pH 7.5** | 1 M | 50 mM | 250 ml |
| **Glycerol** | 87% | 5% | 288 ml |
| **MgCl₂** | 1M | 1.5 mM | 7.5 ml |
| **NaCl** | 5M | 150 mM | 150 ml |
| **Na₃VO₄** | 100 mM | 1mM | 50 ml |

The 5x-DP buffer was filtered through 0.22 µm filter and stored in 40 ml-aliquots at -80°C. These solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

### The following 1x DP buffers were prepared:

- 1x DP buffer (for bead equilibration)
- 1x DP buffer/0.4% NP40 (for bead equilibration and first wash step of beads)
- 1x DP buffer/0.2% NP40 (for second wash step of beads and for compound elution)
- 1x DP buffer/protease inhibitors (for first lysate dilution step); add one protease inhibitor tablet per 25 ml lysis buffer (EDTA-free tablet, protease inhibitor cocktail, Roche Diagnostics, 1 873 580)
- 1x DP buffer/0.4% NP40/protease inhibitors (for second lysate dilution step)

### Example for preparation of 1x DP-buffer/0.4% NP40 (100 ml):

Combine the following solutions and reagents and add distilled water up to a final volume of 100 ml: 20 ml 5x DP buffer, 5 ml 0.5 M NaF, 2 ml 20% NP40, 100 µl 1 M DTT, and add distilled water up to 100 ml. All buffers contain 1 mM DTT final concentration.

### 2.1.2 Washing and equilibration of beads

The kinobeads (Example 1) were prepared for the binding reaction by washing with a suitable buffer and equilibration in the buffer.

The following steps were carried out:
1. Use 15 ml Falcon tubes for all washing steps.
2. Use 100 µl KinoBeads per experiment (settled bead volume): mix equal amounts (25 µl) of each bead type coupled with the following 4 ligands (coupling density of 1 µmol/ml): Bis VIII (CZC00001056), Purvalanol B (CZC00007097), PD173955 derivative (CZC00007324), and CZC00008004.
3. Wash beads two times with 3 ml 1x DP buffer and once with 3 ml 1x DP buffer/0.4% NP40. During each wash step invert tubes 3-5 times, centrifuge 2 minutes at 1200 rpm at 4°C in a Heraeus centrifuge. Supernatants are aspirated supernatants and discarded.

After the last washing step prepare a 1:1 slurry (volume/volume) with 1x DP buffer/0.4% NP40.

### 2.1.3 Preparation of diluted cell lysate

The cell lysate as described under section (1.2) was prepared for the binding reaction by dilution in a suitable buffer and clearing through a centrifugation step. The following steps were carried out:
1. Use a volume of cell lysate corresponding to 50 mg protein per experiment.
2. Thaw the lysate quickly in a 37°C water bath, then keep the sample on ice.
3. Dilute the lysate in the following way:
   1) dilute lysate with 1x DP buffer/protease inhibitors to reduce detergent concentration from 0.8% to 0.4% NP-40.
   2) dilute lysate further with 1x DP buffer/0.4% NP40/protease inhibitors to reach a final protein concentration of 5 mg/ml.
4. Transfer diluted lysate into UZ-polycarbonate tube (Beckmann, 355654).
5. Clear diluted lysate through ultracentrifugation (20 min, 4°C, 100.000 g, TI50.2 rotor, precooled ultracentrifuge).
6. Save supernatant and keep it on ice.

### 2.1.4 Binding reaction and washing

The washed and equilibrated beads from section (2.1.2) were contaced with the diluted cell lysate from step (2.1.3) in order to allow binding of proteins to the ligands. Non-specifically bound proteins were removed by gentle washing in order to reduce background binding.
1. Combine diluted cleared lysate with 100 µl of washed KinoBeads in 15 ml or 50 ml Falcon tube.
2. Incubate for 2 hours at 4°C, rotate on ROTO SHAKE GENIE (Scientific Industries, Inc.) in cold room.
3. After incubation centrifuge for 3 minutes at 1200 rpm in a Heraeus centrifuge or equivalent at 4°C.
4. Remove supernatant carefully without loosing the beads.
5. Transfer the beads to a Mobicol-columns with 90 µm filter (MoBiTec, Goettingen, Cat.no: M1002-90).
6. Wash beads with 10 **ml 1x** DP buffer/0.4% NP-40 and the 5 **ml 1x** DP buffer/0.2% NP-40.
7. Let washing buffer run through the column completely before proceeding with next step
8. Place column in Eppendorf tube and centrifuge them for 1 minute at 800 rpm at 4°C.

Close columns with lower lid.

### 2.1.5 Elution of proteins

1. Add 60 µl 2x NuPAGE SDS Sample Buffer (Invitrogen, NP0007; dilute 4x buffer 1;1 with distilelled water before use).
2. Incubate samples for 30 minutes at 50°C.
3. Open lower lid of column and centrifuge MobiTec columns 1 minute at 2000 rpm to separate eluate from beads.
4. Add 1/10 volume of 200 mg/ml iodoacetamide, incubate for 30 minutes at room temperature, protect from light. This reaction leads to the alkylation of cysteines for mass spectrometry analysis.
5. Before loading samples onto the gel, centrifuge samples for 5 minutes at 15.000 rpm in order to remove precipitates.
6. For protein separation apply □ 60 µl sample to NuPAGE 4-12% Bis-Tris gel (Invitrogen, NP0335).

### 2.1.6 Preparation of stock solutions used in this protocol

### Preparation of a 100 mM Na₃VO₄ stock solution:

1) Dissolve 9.2 g Na₃VO₄ in 400 ml distilled water.
2) Adjust the pH to 10.0 using either 1 N NaOH or 1 N HCl. The starting pH of the sodium orthovanadate solution may vary with batch. At pH 10.0 the solution will be yellow.
3) Boil the solution until it turns colorless (approximately 10 min).
4) Cool to room temperature.
5) Readjust the pH to 10.0 and repeat steps 2 and 3 until solution remains colorless and the pH stabilizes at 10.0.
6) Adjust the volume to 500 ml with distilled water.
7) Freeze aliquots at -20°C. Aliquots can be stored for several months.

### Preparation of a 500 mM NaF stock solution:

Dissolve 21.0 g NaF (Sigma, S7920) in 500 ml distilled water. Filter solution through a 0.22 µm filter and store at 4°C.

### Preparation of a 20% NP40-solution:

Weigh 40.0 g NP40 (Sigma, Igepal-CA630, cat.no. 13021). Add distilled water up to 200 g. Mix completely and store solution at room temperature.

### Preparation of a 1 M DTT solution:

Dissolve 7.7 g DTT (Biomol, catalogue number 04010) in 50 ml distilled water. Filter solution through 0.22 µm filter and freeze 400 µl aliquots at -20°C. Aliquots can be stored for several months.

### Preparation of a Iodoacetamide stock solution (200 mg/ml):

Dissolve 2.0 g Iodoacetamide (Sigma, I-6125) in 10 ml distilled water. Filter solution through 0.22 µm filter and freeze 400 µl aliquots at -20°C. Aliquots can be stored for several months.

### 2.2 Immunoprecipitation using anti-phosphotyrosine antibody beads

### 2.2.1 Buffers and Antiphosphotyrosine antibody beads

All buffers used in the imunoprecipitation experiment with immobilized anti-phosphotyrosine antibodies were identical to those used for the kinobead experiment (see above) except that no DTT was added and 50 µL 1mM okadaic acid (Biomol, El-181; phosphatase inhibitor) was added so that a final concentration of 500 nM okadaic acid was reached. Antiphosphotyrosine antibody beads (Agarose beads with covalently coupled recombinant 4G10 anti-phosphotyrosine antibody) were obtained from Biomol (Catalogue number 16-199).

### 2.2.2 Washing and equilibration of anti-phosphotyrosine beads

The anti-phosphotyrosine beads were prepared for the binding reaction by washing with a suitable buffer and equilibration in the buffer.
1. Use 15 ml Falcon tubes for all washing steps.
2. Use 100 µl anti-phosphotyrosine beads per experiment.
3. Wash beads two times with 3 ml 1x DP buffer (-DTT) and once with 3 ml 1x DP buffer/0.4% NP40 (-DTT). During each wash step invert tubes 3-5 times, centrifuge 2 minutes at 1200 rpm at 4°C in a Heraeus centrifuge. Supernatants are aspirated and discarded.

After the last washing step prepare a 1:1 slurry (volume/volume) with 1x DP buffer/0.4% NP40 (-DTT).

### 2.2.3 Preparation of diluted cell lysate

The cell lysate was prepared for the binding reaction by dilution in a suitable buffer and clearing through a centrifugation step.
1. Use a volume of cell lysate corresponding to 50 mg protein per experiment.
2. Thaw the lysate quickly in a 37°C water bath, then keep the sample on ice.
3. Dilute the lysate in the following way:
   First dilution step: dilute lysate with 1x DP buffer/protease inhibitors/ okadaic acid /without DTT to reduce detergent concentration from 0.8% to 0.4% NP-40.
   Second dilution step: dilute lysate further with 1x DP buffer/0.4% NP40/protease inhibitors/okadaic acid / without DTT to reach a final protein concentration of 5 mg/ml.
   (Note: The second dilution step is only required if the protein concentration of the lysate after the first dilution step is higher than 5 mg/ml).
4. Transfer diluted lysate into UZ-polycarbonate tube (Beckmann, 355654).
5. Clear diluted lysate through ultracentrifugation (20 min, 4°C, 100.000 g, TI50.2 rotor, precooled ultracentrifuge).
6. Save supernatant and keep it on ice.

### 2.2.4 Binding reaction and washing steps

The washed and equilibrated anti-phosphotyrosine beads were contaced with the diluted cell lysate from section 2.2.3 in order to allow binding of proteins to the anti-phosphotyrosine beads. Non-specifically bound proteins were removed by gentle washing in order to reduce background binding.
1. Combine diluted cleared lysate with 100 µl of washed anti-phosphotyrosine beads in a 15 ml or 50 ml Falcon tube.
2. Incubate for 4 hours at 4°C, rotate on ROTO SHAKE GENIE (Scientific Industries, Inc.) in the cold room.
3. After incubation centrifuge for 3 minutes at 1200 rpm in a Heraeus centrifuge or equivalent at 4°C.
4. Remove supernatant carefully without loosing the beads.
5. Transfer the beads to a Mobicol-column with 90 µm filter (MoBiTec, Goettingen, Cat.no: M1002-90).
6. Wash beads with 10 **ml 1x** DP buffer/0.4% NP-40/without DTT and 5 **ml 1x** DP buffer/0.2% NP-40/without DTT.
7. Let washing buffer run through the column completely before proceeding with next step.
8. Place column in Eppendorf tube and centrifuge them for 1 minute at 800 rpm at 4°C.

Close columns with lower lid.

### 2.2.5 Elution of bound proteins

1. Add 60 µl 2x NuPAGE SDS Sample Buffer (Invitrogen, NP0007; dilute 4x buffer 1;1 with distilelled water before use).
2. Incubate samples for 30 minutes at 50°C.
3. Open lower lid of column and centrifuge MobiTec columns 1 minute at 2000 rpm to separate eluate from beads.
4. Add 1/10 volume of 200 mg/ml iodoacetamide, incubate for 30 minutes at room temperature, protect from light. This reaction leads to the alkylation of cysteines for mass spectrometry analysis.
5. Before loading samples onto the gel, centrifuge samples for 5 minutes at 15.000 rpm in order to remove precipitates.
6. For protein separation apply □ 60 µl sample to NuPAGE 4-12% Bis-Tris gel (Invitrogen, NP0335).

### 3. Mass spectrometric analysis of eluted enzymes (e.g. kinases)

### Description of proteotypic peptides

Tryptic digestion of a SDS-PAGE-separated protein mixture generates for each protein numerous distinct peptide fragments with different physico-chemical properties. These peptides differ in compatibility with the mass spectrometry-based analytical platform used for protein identification (ID), here nanocapillary reversed phase-liquid chromatography electrospray ionization tandem mass spectrometry (RP-LC-MS/MS). As a result, peptide frequencies for peptides from the same source protein differ by a great degree, the most frequently observed peptides that "typically" contribute to the identification of this protein being termed "proteotypic" peptides. Thus, a "proteotypic peptide" is an experimentally well observable peptide that uniquely identifies a specific protein or protein isoform.

### Advantages of proteotypic peptides

The use of proteotypic peptides for protein identification allows rapid and focussed identification and quantitation of multiple known target proteins by focusing the protein identification process on a screening for the presence of information-rich signature peptides.

### Experimental identification of proteotypic peptides

One strategy to generate a list of proteotypic peptides is to collect peptide-identification data empirically and to search the dataset for commonly observed peptides that uniquely identify a protein.

For each IPI database protein entry with at least 10 unequivocal identifications in the CZ dataset (multipeptide IDs or manually verified single peptide IDs), peptide frequencies for contributing peptides are calculated. Only specific, best peptide-to-spectrum matches according to the database search engine Mascot™ (Matrix Science) are considered.

For definition of proteotypic peptides for a specific protein, peptides are ordered by descending peptide frequency and a cumulative peptide presence is calculated: This value gives for each peptide the ratio of identifications where this peptide or any of the peptides with a higher peptide frequency was present. Proteotypic peptides are defined using a cutoff for cumulative presence of 95%, i.e. at least 95% of identification events for this protein were based on at least one proteotypic peptide.

### 3.1 Protein digestion and sample preparation prior to mass spectrometric analysis

Proteins were concentrated, separated on 4-12% NuPAGE® Novex gels (Invitrogen, Carlsbad, CA), and stained with colloidal Coomassie blue. Gel lanes were systematically cut across the entire separation range into ≤ 48 slices (bands and interband regions) and subjected to in-gel tryptic digestion essentially as described by Shevchenko et al., 1996, Analytical Chemistry 68: 850-858. Briefly, gel plugs were destained overnight in 5mM NH₄HCO₃ in 50%EtOH, digested with for 4 hours with trypsin at 12.5 ng/µl in 5mM NH₄HCO₃. Peptides were extracted with 1% formic acid, transferred into a second 96 well plate and dried under vacuum. Dry peptides were resuspended 10 µl 0.1 % formic acid in water and 5 µl were injected into the LC-MS/MS system for protein identification.

### 3.2 Mass spectrometric data acquisition

Peptide samples were injected into a nano LC system (CapLC, Waters or Ultimate, Dionex) which was directly coupled either to a quadrupole time-of-fligth (QTOF2, QTOF Ultima, QTOF Micro, Micromass or QSTAR Pulsar, Sciex) or ion trap (LCQ Deca XP, LTQ, Thermo-Finnigan) mass spectrometer. Peptides were separated on the LC system using a gradient of aqueous and organic solvents with a typical gradient time of between 15 and 45 min. Solvent A was 5% acetonitrile in 0.1% formic acid and solvent B was 70% acetonitrile in 0.1 % formic acid.

### 3.3 Protein identification

The peptide mass and fragmentation data generated in the LC-MS/MS experiments were used to query an in-house curated version of the International Protein Index (IPI) protein sequence database (EBI) Proteins were identified by correlating the measured peptide mass and fragmentation data with the same data computed from the entries in the database using the software tool Mascot (Matrix Science; Perkins et al., 1999, Electrophoresis 20: 3551-3567). Search criteria varied depending on which mass spectrometer was used for the analysis.

### 4. Results

The results of the Signalokinome experiment are shown in Figure 2. This figure shows a comparison of Drug pulldowns using kinobeads (left circle) and conventional imunoprecipitations with anti-phosphotyrosine antibody beads (right circle). In the Kinobeads experiment a total of 626 proteins were identified, of these were 100 kinases. In the immunoprecipitation (IP) experiment a total of 503 proteins were identified, 12 of these were kinases. Kinases identified with both experiments (common kinases) are listed in the overlap area. The result shows that with the kinobeads significantly more kinases were identified (100 kinases) compared to the anti-phosphotyrosine antibody beads (12 kinases).

The identified kinases for both experimental approaches are listed in the following tables. In addition, the sequences of proteotypic peptides for the kinases are listed in separate tables.

**Table 6: Kinases identified after immunoprecipitation (IP). Kinase names are in accordance with the human kinase nomenclature (http://kinase.com) and Manning et al., 2002, Science 298, 1912-1934 as specified in supplementary material).**

| **Kinase identification** | **Kinase Name** | **Score** | **Number of identified peptides** | **Experimental proteotypic peptide available and identified** |
|---|---|---|---|---|
| 1 | DNAPK | 606 | 20 | X |
| 2 | TIF1b | 202 | 6 | X |
| 3 | p38a | 343 | 11 | X |
| 4 | CDK2 | 196 | 4 | X |
| 5 | TYK2 | 77 | 3 | X |
| 6 | LYN | 67 | 2 | X |
| 7 | EGFR | 2463 | 162 | X |
| 8 | FAK | 1203 | 51 | X |
| 9 | ACK | 160 | 6 | |
| 10 | KHS2 | 65 | 2 | |

**Table 7: Sequences of proteotypic peptides for kinases identified after immunoprecipitation (IP, see Table 6). Kinase names are in accordance with the human kinase nomenclature (http://kinase.com) and Manning et al., 2002, Science 298, 1912-1934 as specified in supplementary material).**

| **EXPERIMENT** | **KINASE NAME** | **IDENTIFIED PROTEOTYPIC PEPTIDE** |
|---|---|---|
| IP | CDK2 | ALFPGDSEIDQLFR |
| IP | CDK2 | IGEGTYGVVYK |
| IP | CDK2 | FMDASALTGIPLPLIK |
| IP | DNAPK | HGDLPDIQIK |
| IP | DNAPK | LACDVDQVTR |
| IP | DNAPK | AALSALESFLK |
| IP | DNAPK | DQNILLGTTYR |
| IP | DNAPK | FMNAVFFLLPK |
| IP | DNAPK | VTELALTASDR |
| IP | DNAPK | LGASLAFNNIYR |
| IP | DNAPK | LNESTFDTQITK |
| IP | DNAPK | QLFSSLFSGILK |
| IP | DNAPK | NILEESLCELVAK |
| IP | DNAPK | TVSLLDENNVSSYLSK |
| IP | DNAPK | TVGALQVLGTEAQSSLLK |
| IP | EGFR | VLGSGAFGTVYK |
| IP | EGFR | IPLENLQIIR |
| IP | EGFR | GDSFTHTPPLDPQELDILK |
| IP | FAK | LGDFGLSR |
| IP | FAK | FLQEALTMR |
| IP | FAK | FFEILSPVYR |
| IP | FAK | LLNSDLGELINK |
| IP | FAK | TLLATVDETIPLLPASTHR |
| IP | HER2/ErbB2 | VLGSGAFGTVYK |
| IP | LYN | EEPIYIITEYMAK |
| IP | p38a | LTDDHVQFLIYQILR |
| IP | p38a | NYIQSLTQMPK |
| IP | p38a | LTGTPPAYLINR |
| IP | TIT1b | IVAERPGTNSTGPAPMAPPR |
| IP | TYK2 | HGIPLEEVAK |
| IP | TYK2 | LSDPGVGLGALSR |

**Table 8: Kinases identified after drug pulldown with kinobeads. Kinase names are in accordance with the human kinase nomenclature (http://kinase.com) and Manning et al., 2002, Science 298, 1912-1934 as specified in supplementary material).**

| **Kinase identification** | **Kinase Name** | **Score** | **Number of peptides identified** | **Experimental proteotypic peptide available and identified** | **Identified also in IP (see table 1)** |
|---|---|---|---|---|---|
| 1 | DNAPK | 6592 | 263 | X | IP |
| 2 | FRAP | 356 | 9 | X | |
| 3 | ATM | 113 | 3 | X | |
| 4 | ATR | 91 | 2 | X | |
| 5 | CDC2 | 309 | 11 | X | |
| 6 | YES | 1747 | 100 | X | |
| 7 | CaMK2d | 1003 | 41 | X | |
| 8 | JNK1 | 1011 | 45 | X | |
| 9 | JNK2 | 317 | 11 | X | |
| 10 | CaMK2g | 811 | 21 | X | |
| 11 | p38a | 1145 | 90 | X | IP |
| 12 | TNK1 | 81 | 4 | X | |
| 13 | GSK3B | 1408 | 81 | X | |
| 14 | FYN | 1845 | 115 | X | |
| 15 | SRC | 1780 | 91 | X | |
| 16 | Erk2 | 1677 | 200 | X | |
| 17 | CaMK2b | 122 | 3 | X | |
| 18 | JNK3 | 590 | 26 | X | |
| 19 | CK2a1 | 316 | 11 | X | |
| 20 | GSK3A | 1390 | 117 | X | |
| 21 | AurA | 198 | 8 | X | |
| 22 | RIPK2 | 634 | 19 | X | |
| 23 | CDK2 | 1172 | 50 | X | IP |
| 24 | ADCK1 | 88 | 4 | | |
| 25 | CK1a | 566 | 21 | X | |
| 26 | GAK | 1995 | 61 | X | |
| 27 | AAK1 | 123 | 3 | X | |
| 28 | CDK9 | 379 | 13 | X | |
| 29 | CK1d | 206 | 7 | X | |
| 30 | Erk1 | 566 | 26 | X | |
| 31 | NEK9 | 1947 | 102 | X | |
| 32 | TYK2 | 222 | 6 | X | IP |
| 33 | AMPKa1 | 93 | 3 | X | |
| 34 | LYN | 1783 | 67 | X | IP |
| 35 | AurB | 287 | 10 | X | |
| 36 | CK1e | 314 | 10 | X | |
| 37 | EGFR | 165 | 3 | X | IP |
| 38 | EphB2 | 1165 | 43 | X | |
| 39 | ALK2 | 299 | 11 | X | |
| 40 | DDR1 | 759 | 28 | X | |
| 41 | TBK1 | 2042 | 72 | X | |
| 42 | CSK | 1584 | 102 | X | |
| 43 | TEC | 50 | 2 | | |
| 44 | ZAK | 499 | 16 | X | |
| 45 | ALK4 | 472 | 9 | X | |
| 46 | ADCK3 | 309 | 8 | X | |
| 47 | MAP2K5 | 795 | 32 | | |
| 48 | EphB4 | 2237 | 165 | X | |
| 49 | ARG | 2128 | 69 | X | |
| 50 | FER | 2762 | 130 | X | |
| 51 | RSK2 | 2100 | 99 | X | |
| 52 | CK2a2 | 930 | 32 | X | |
| 53 | BMPR1A | 676 | 19 | X | |
| 54 | TGFbR1 | 478 | 15 | X | |
| 55 | EphA5 | 114 | 4 | X | |
| 56 | BRK | 107 | 3 | | |
| 57 | FAK | 1933 | 95 | X | IP |
| 58 | ILK | 209 | 6 | X | |
| 59 | CDK5 | 699 | 27 | X | |
| 60 | DDR2 | 1073 | 44 | X | |
| 61 | JAK1 | 1083 | 31 | | |
| 62 | MYT1 | 152 | 5 | | |
| 63 | PKCd | 53 | 4 | | |
| 64 | RSK3 | 2227 | 117 | X | |
| 65 | Wee1 | 550 | 18 | X | |
| 66 | ACTR2 | 193 | 5 | | |
| 67 | MAP3K2 | 383 | 15 | | |
| 68 | ACTR2B | 82 | 2 | | |
| 69 | Wee1B | 550 | 18 | X | |
| 70 | ABL | 1133 | 35 | X | |
| 71 | MET | 503 | 12 | X | |
| 72 | BRAF | 361 | 7 | | |
| 73 | INSR | 1807 | 64 | X | |
| 74 | KHS1 | 393 | 12 | | |
| 75 | PAK4 | 448 | 15 | X | |
| 76 | PKD1 | 58 | 2 | | |
| 77 | PKD2 | 58 | 2 | X | |
| 78 | IGF1R | 935 | 28 | X | |
| 79 | PHKg2 | 519 | 16 | X | |
| 80 | CaMKK2 | 199 | 5 | X | |
| 81 | MAP2K2 | 294 | 6 | X | |
| 82 | TGFbR2 | 49 | 2 | | |
| 83 | Fused | 42 | 2 | | |
| 84 | EphA2 | 2099 | 90 | X | |
| 85 | ACK | 169 | 4 | | IP |
| 86 | NLK | 46 | 2 | | |
| 87 | CDK7 | 623 | 20 | X | |
| 88 | CHK2 | 58 | 2 | X | |
| 89 | KHS2 | 60 | 4 | | IP |
| 90 | MLK3 | 59 | 1 | X | |
| 91 | PKCe | 53 | 4 | X | |
| 92 | PKCh | 53 | 4 | X | |
| 93 | PYK2 | 106 | 3 | X | |
| 94 | TA02 | 120 | 3 | | |
| 95 | CK1g1 | 45 | 2 | X | |
| 96 | LIMK1 | 271 | 6 | | |
| 97 | MAP2K1 | 480 | 14 | | |
| 98 | HRI | 52 | 1 | | |
| 99 | SIK | 36 | 1 | | |
| 100 | Erk7 | 67 | 1 | | |
| 101 | NEK2 | 145 | 4 | | |
| 102 | NEK7 | 175 | 5 | | |
| 103 | BMPR1B | 179 | 5 | X | |
| 104 | MAP2K3 | 133 | 5 | | |
| 105 | FGFR2 | 514 | 17 | | |

**Table 9: Sequences of proteotypic peptides for kinases identified after drug pulldowns with kinobeads (see Table 8)**

| **EXPERIMENT** | **KINASE NAME** | **IDENTIFIED PROTEOTYPIC PEPTIDE** |
|---|---|---|
| KinobeadDP | AAK1 | ADIWALGCLLYK |
| KinobeadDP | ABL | GQGESDPLDHEPAVSPLLPR |
| KinobeadDP | ABL | WTAPESLAYNK |
| KinobeadDP | ABL | VADFGLSR |
| KinobeadDP | ABL | NGQGWVPSNYITPVNSLEK |
| KinobeadDP | ADCK3 | DKLEYFEERPFAAASIGQVHLAR |
| KinobeadDP | ADCK3 | SFTDLYIQIIR |
| KinobeadDP | ADCK3 | VALLDFGATR |
| KinobeadDP | ADCK3 | AVLGSSPFLSEANAER |
| KinobeadDP | ALK2 | WFSDPTLTSLAK |
| KinobeadDP | ALK4 | TIVLQEIIGK |
| KinobeadDP | ALK4 | EAEIYQTVMLR |
| KinobeadDP | AMPKa1 | IGHYILGDTLGVGTFGK |
| KinobeadDP | ARG | AASSSSVVPYLPR |
| KinobeadDP | ARG | ESESSPGQLSISLR |
| KinobeadDP | ARG | GAQASSGSPALPR |
| KinobeadDP | ARG | VLGYNQNGEWSEVR |
| KinobeadDP | ARG | VPVLISPTLK |
| KinobeadDP | ARG | WTAPESLAYNTFSIK |
| KinobeadDP | ARG | VADFGLSR |
| KinobeadDP | ARG | NGQGWVPSNYITPVNSLEK |
| KinobeadDP | ATM | LVVNLLQLSK |
| KinobeadDP | ATR | APLNETGEVVNEK |
| KinobeadDP | AurA | SKQPLPSAPENNPEEELASK |
| KinobeadDP | AurA | VEFTFPDFVTEGAR |
| KinobeadDP | AurB | IYLILEYAPR |
| KinobeadDP | AurB | SNVQPTAAPGQK |
| KinobeadDP | BMPR1A | FNSDTNEVDVPLNTR |
| KinobeadDP | BMPR1A | WNSDECLR |
| KinobeadDP | BMPR1A | YEGSDFQCK |
| KinobeadDP | BMPR1A | YMAPEVLDESLNK |
| KinobeadDP | BMPR1A | ETEIYQTVLMR |
| KinobeadDP | BMPR1A | HENILGFIAADIK |
| KinobeadDP | BMPR1A | VFFTTEEASWFR |
| KinobeadDP | BMPR1A | YGEVWMGK |
| KinobeadDP | BMPR1B | ETEIYQTVLMR |
| KinobeadDP | BMPR1B | HENILGFIAADIK |
| KinobeadDP | BMPR1B | VFFTTEEASWFR |
| KinobeadDP | BMPR1B | YGEVWMGK |
| KinobeadDP | CaMK2b | LTQYIDGQGRPR |
| KinobeadDP | CaMK2b | NLINQMLTINPAK |
| KinobeadDP | CaMK2b | AGAYDFPSPEWDTVTPEAK |
| KinobeadDP | CaMK2b | DLKPENLLLASK |
| KinobeadDP | CaMK2d | FYFENALSK |
| KinobeadDP | CaMK2d | AGAYDFPSPEWDTVTPEAK |
| KinobeadDP | CaMK2d | DLKPENLLLASK |
| KinobeadDP | CaMK2g | FYFENLLSK |
| KinobeadDP | CaMK2g | LTQYIDGQGRPR |
| KinobeadDP | CaMK2g | NLINQMLTINPAK |
| KinobeadDP | CaMK2g | AGAYDFPSPEWDTVTPEAK |
| KinobeadDP | CaMK2g | DLKPENLLLASK |
| KinobeadDP | CaMKK2 | GPIEQVYQEIAILK |
| KinobeadDP | CaMKK2 | LAYNENDNTYYAMK |
| KinobeadDP | CDC2 | DLKPQNLLIDDKGTIK |
| KinobeadDP | CDC2 | NLDENGLDLLSK |
| KinobeadDP | CDC2 | SPEVLLGSAR |
| KinobeadDP | CDC2 | IGEGTYGVVYK |
| KinobeadDP | CDK2 | APEILLGCK |
| KinobeadDP | CDK2 | FMDASALTGIPLPLIK |
| KinobeadDP | CDK2 | ALFPGDSEIDQLFR |
| KinobeadDP | CDK2 | IGEGTYGVVYK |
| KinobeadDP | CDK5 | IGEGTYGTVFK |
| KinobeadDP | CDK5 | SFLFQLLK |
| KinobeadDP | CDK7 | APELLFGAR |
| KinobeadDP | CDK7 | VPFLPGDSDLDQLTR |
| KinobeadDP | CDK9 | GSQITQQSTNQSR |
| KinobeadDP | CDK9 | IGQGTFGEVFK |
| KinobeadDP | CK1a | HPQLLYESK |
| KinobeadDP | CK1a | LFLIDFGLAK |
| KinobeadDP | CK1d | FDDKPDYSYLR |
| KinobeadDP | CK1d | GNLVYIIDFGLAK |
| KinobeadDP | CK1d | TVLLLADQMISR |
| KinobeadDP | CK1e | FDDKPDYSYLR |
| KinobeadDP | CK1e | GNLVYIIDFGLAK |
| KinobeadDP | CK1e | TVLLLADQMISR |
| KinobeadDP | CK1g1 | TLFTDLFEK |
| KinobeadDP | CK2a1 | GGPNIITLADIVKDPVSR |
| KinobeadDP | CK2a1 | QLYQTLTDYDIR |
| KinobeadDP | CK2a1 | TPALVFEHVNNTDFK |
| KinobeadDP | CK2a2 | HLVSPEALDLLDKLLR |
| KinobeadDP | CK2a2 | LIDWGLAEFYHPAQEYNVR |
| KinobeadDP | CK2a2 | QLYQILTDFDIR |
| KinobeadDP | CK2a2 | TPALVFEYINNTDFK |
| KinobeadDP | CK2a2 | VLGTEELYGYLK |
| KinobeadDP | CSK | HSNLVQLLGVIVEEK |
| KinobeadDP | CSK | LLYPPETGLFLVR |
| KinobeadDP | CSK | VMEGTVAAQDEFYR |
| KinobeadDP | DDR1 | AQPFGQLTDEQVIENAGEFFR |
| KinobeadDP | DDR1 | NCLVGENFTIK |
| KinobeadDP | DDR1 | NLYAGDYYR |
| KinobeadDP | DDR1 | QVYLSRPPACPQGLYELMLR |
| KinobeadDP | DDR1 | WMAWECILMGK |
| KinobeadDP | DDR1 | YLATLNFVHR |
| KinobeadDP | DDR2 | DVAVEEFPR |
| KinobeadDP | DDR2 | FMATQIASGMK |
| KinobeadDP | DDR2 | HEPPNSSSSDVR |
| KinobeadDP | DDR2 | IFPLRPDYQEPSR |
| KinobeadDP | DDR2 | NLYSGDYYR |
| KinobeadDP | DDR2 | QTYLPQPAICPDSVYK |
| KinobeadDP | DDR2 | WMSWESILLGK |
| KinobeadDP | DDR2 | YLSSLNFVHR |
| KinobeadDP | DNAPK | AALSALESFLK |
| KinobeadDP | DNAPK | DQNILLGTTYR |
| KinobeadDP | DNAPK | FMNAVFFLLPK |
| KinobeadDP | DNAPK | FVPLLPGNR |
| KinobeadDP | DNAPK | GLSSLLCNFTK |
| KinobeadDP | DNAPK | HGDLPDIQIK |
| KinobeadDP | DNAPK | INQVFHGSCITEGNELTK |
| KinobeadDP | DNAPK | IPALDLLIK |
| KinobeadDP | DNAPK | LACDVDQVTR |
| KinobeadDP | DNAPK | LAGANPAVITCDELLLGHEK |
| KinobeadDP | DNAPK | LGASLAFNNIYR |
| KinobeadDP | DNAPK | LGNPIVPLNIR |
| KinobeadDP | DNAPK | LLEEALLR |
| KinobeadDP | DNAPK | LNESTFDTQITK |
| KinobeadDP | DNAPK | LPLISGFYK |
| KinobeadDP | DNAPK | LPSNTLDR |
| KinobeadDP | DNAPK | LQETLSAADR |
| KinobeadDP | DNAPK | MSTSPEAFLALR |
| KinobeadDP | DNAPK | NILEESLCELVAK |
| KinobeadDP | DNAPK | NLLIFENLIDLK |
| KinobeadDP | DNAPK | NLSSNEAISLEEIR |
| KinobeadDP | DNAPK | QITQSALLAEAR |
| KinobeadDP | DNAPK | QLFSSLFSGILK |
| KinobeadDP | DNAPK | SLGPPQGEEDSVPR |
| KinobeadDP | DNAPK | SQGCSEQVLTVLK |
| KinobeadDP | DNAPK | TVGALQVLGTEAQSSLLK |
| KinobeadDP | DNAPK | TVSLLDENNVSSYLSK |
| KinobeadDP | DNAPK | VCLDIIYK |
| KinobeadDP | DNAPK | VTELALTASDR |
| KinobeadDP | DNAPK | VVQMLGSLGGQINK |
| KinobeadDP | EGFR | GDSFTHTPPLDPQELDILK |
| KinobeadDP | EphA2 | DGEFSVLQLVGMLR |
| KinobeadDP | EphA2 | FADIVSILDK |
| KinobeadDP | EphA2 | LPSTSGSEGVPFR |
| KinobeadDP | EphA2 | NILVNSNLVCK |
| KinobeadDP | EphA2 | VSDFGLSR |
| KinobeadDP | EphA5 | WTAPEAIAFR |
| KinobeadDP | EphA5 | VSDFGLSR |
| KinobeadDP | EphB2 | AGAIYVFQVR |
| KinobeadDP | EphB2 | FGQIVNTLDK |
| KinobeadDP | EphB2 | TFPNWMENPWVK |
| KinobeadDP | EphB2 | VDTIAADESFSQVDLGGR |
| KinobeadDP | EphB2 | NILVNSNLVCK |
| KinobeadDP | EphB2 | VSDFGLSR |
| KinobeadDP | EphB4 | APSGAVLDYEVK |
| KinobeadDP | EphB4 | FPQVVSALDK |
| KinobeadDP | EphB4 | LNDGQFTVIQLVGMLR |
| KinobeadDP | EphB4 | WTAPEAIAFR |
| KinobeadDP | EphB4 | NILVNSNLVCK |
| KinobeadDP | EphB4 | VSDFGLSR |
| KinobeadDP | Erk1 | NYLQSLPSK |
| KinobeadDP | Erk1 | APEIMLNSK |
| KinobeadDP | Erk1 | YIHSANVLHR |
| KinobeadDP | Erk1 | ICDFGLAR |
| KinobeadDP | Erk2 | FRHENIIGINDIIR |
| KinobeadDP | Erk2 | GQVFDVGPR |
| KinobeadDP | Erk2 | LKELIFEETAR |
| KinobeadDP | Erk2 | NYLLSLPHK |
| KinobeadDP | Erk2 | VADPDHDHTGFLTEYVATR |
| KinobeadDP | Erk2 | APEIMLNSK |
| KinobeadDP | Erk2 | YIHSANVLHR |
| KinobeadDP | Erk2 | ICDFGLAR |
| KinobeadDP | FAK | FFEILSPVYR |
| KinobeadDP | FAK | FLQEALTMR |
| KinobeadDP | FAK | LLNSDLGELINK |
| KinobeadDP | FAK | TLLATVDETIPLLPASTHR |
| KinobeadDP | FAK | LGDFGLSR |
| KinobeadDP | FER | LQDWELR |
| KinobeadDP | FER | QEDGGVYSSSGLK |
| KinobeadDP | FER | SGVVLLNPIPK |
| KinobeadDP | FER | WTAPEALNYGR |
| KinobeadDP | FRAP | GPTPAILESLISINNK |
| KinobeadDP | FRAP | VLGLLGALDPYK |
| KinobeadDP | FYN | EVLEQVER |
| KinobeadDP | FYN | WTAPEAALYGR |
| KinobeadDP | FYN | LIEDNEYTAR |
| KinobeadDP | FYN | GSLLDFLK |
| KinobeadDP | FYN | IADFGLAR |
| KinobeadDP | GAK | ALVEEEITR |
| KinobeadDP | GAK | AMLQVNPEER |
| KinobeadDP | GAK | AVVMTPVPLFSK |
| KinobeadDP | GAK | IAVMSFPAEGVESALK |
| KinobeadDP | GAK | TPEIIDLYSNFPIGEK |
| KinobeadDP | GSK3A | VIGNGSFGVVYQAR |
| KinobeadDP | GSK3A | VTTVVATLGQGPER |
| KinobeadDP | GSK3A | YFFYSSGEK |
| KinobeadDP | GSK3B | LLEYTPTAR |
| KinobeadDP | GSK3B | LYMYQLFR |
| KinobeadDP | GSK3B | VIGNGSFGVVYQAK |
| KinobeadDP | GSK3B | YFFYSSGEK |
| KinobeadDP | IGF1R | AENGPGPGVLVLR |
| KinobeadDP | IGF1R | HSHALVSLSFLK |
| KinobeadDP | IGF1R | MYFAFNPK |
| KinobeadDP | IGF1R | TTINNEYNYR |
| KinobeadDP | IGF1R | VAGLESLGDLFPNLTVIR |
| KinobeadDP | IGF1R | DIYETDYYR |
| KinobeadDP | IGF1R | IEFLNEASVMK |
| KinobeadDP | IGF1R | IGDFGMTR |
| KinobeadDP | ILK | MYAPAWVAPEALQK |
| KinobeadDP | INSR | DLLGFMLFYK |
| KinobeadDP | INSR | ELGQGSFGMVYEGNAR |
| KinobeadDP | INSR | ESLVISGLR |
| KinobeadDP | INSR | TIDSVTSAQELR |
| KinobeadDP | INSR | TVNESASLR |
| KinobeadDP | INSR | WEPYWPPDFR |
| KinobeadDP | INSR | DIYETDYYR |
| KinobeadDP | INSR | IEFLNEASVMK |
| KinobeadDP | INSR | WMAPESLK |
| KinobeadDP | INSR | IGDFGMTR |
| KinobeadDP | JNK1 | NIIGLLNVFTPQK |
| KinobeadDP | JNK1 | APEVILGMGYK |
| KinobeadDP | JNK1 | ILDFGLAR |
| KinobeadDP | JNK2 | NIISLLNVFTPQK |
| KinobeadDP | JNK2 | APEVILGMGYK |
| KinobeadDP | JNK2 | ILDFGLAR |
| KinobeadDP | JNK3 | NIISLLNVFTPQK |
| KinobeadDP | JNK3 | APEVILGMGYK |
| KinobeadDP | JNK3 | ILDFGLAR |
| KinobeadDP | LYN | EEPIYIITEYMAK |
| KinobeadDP | LYN | GSLLDFLK |
| KinobeadDP | LYN | IADFGLAR |
| KinobeadDP | MAP2K2 | ISELGAGNGGVVTK |
| KinobeadDP | MAP2K2 | YPIPPPDAK |
| KinobeadDP | MET | AFFMLDGILSK |
| KinobeadDP | MET | DLIGFGLQVAK |
| KinobeadDP | MLK3 | ITVQASPGLDR |
| KinobeadDP | NEK9 | AGGGAAEQEELHYIPIR |
| KinobeadDP | NEK9 | LGINLLGGPLGGK |
| KinobeadDP | NEK9 | LQQENLQIFTQLQK |
| KinobeadDP | NEK9 | SSTVTEAPIAVVTSR |
| KinobeadDP | NEK9 | TSEVYVWGGGK |
| KinobeadDP | p38a | LTGTPPAYLINR |
| KinobeadDP | p38a | NYIQSLTQMPK |
| KinobeadDP | p38a | SLEEFNDVYLVTHLMGADLNNIVK |
| KinobeadDP | p38a | LTDDHVQFLIYQILR |
| KinobeadDP | p38a | ILDFGLAR |
| KinobeadDP | PAK4 | AALQLVVDPGDPR |
| KinobeadDP | PHKg2 | LTAEQALQHPFFER |
| KinobeadDP | PHKg2 | SLLEAVSFLHANNIVHR |
| KinobeadDP | PHKg2 | VAVWTVLAAGR |
| KinobeadDP | PHKg2 | LSPEQLEEVR |
| KinobeadDP | PYK2 | EIITSILLSGR |
| KinobeadDP | PYK2 | EVGLDLFFPK |
| KinobeadDP | PYK2 | VLANLAHPPAE |
| KinobeadDP | PYK2 | LGDFGLSR |
| KinobeadDP | RIPK2 | CLIELEPVLR |
| KinobeadDP | RIPK2 | SLPAPQDNDFLSR |
| KinobeadDP | RIPK2 | SPSLNLLQNK |
| KinobeadDP | RIPK2 | TQNILLDNEFHVK |
| KinobeadDP | RSK2 | EASAVLFTITK |
| KinobeadDP | RSK2 | LGMPQFLSPEAQSLLR |
| KinobeadDP | RSK2 | NQSPVLEPVGR |
| KinobeadDP | RSK2 | LYLILDFLR |
| KinobeadDP | RSK3 | APQAPLHSVVQQLHGK |
| KinobeadDP | RSK3 | EASFVLHTIGK |
| KinobeadDP | RSK3 | LGMPQFLSTEAQSLLR |
| KinobeadDP | RSK3 | LYLILDFLR |
| KinobeadDP | SRC | AANILVGENLVCK |
| KinobeadDP | SRC | WTAPEAALYGR |
| KinobeadDP | SRC | LIEDNEYTAR |
| KinobeadDP | SRC | GSLLDFLK |
| KinobeadDP | TBK1 | EPLNTIGLIYEK |
| KinobeadDP | TBK1 | FGSLTMDGGLR |
| KinobeadDP | TBK1 | IISSNQELIYEGR |
| KinobeadDP | TBK1 | LFAIEEETTTR |
| KinobeadDP | TBK1 | TTEENPIFVVSR |
| KinobeadDP | TBK1 | VIGEDGQSVYK |
| KinobeadDP | TGFbR1 | HDSATDTIDIAPNHR |
| KinobeadDP | TGFbR1 | TIVLQESIGK |
| KinobeadDP | TGFbR1 | TLSQLSQQEGIK |
| KinobeadDP | TGFbR1 | VPNEEDPSLDRPFISEGTTLK |
| KinobeadDP | TGFbR1 | EAEIYQTVMLR |
| KinobeadDP | TNK1 | MLPEAGSLWLLK |
| KinobeadDP | TYK2 | LGLAEGTSPFIK |
| KinobeadDP | Wee1B | IPQVLSQEFTELLK |
| KinobeadDP | Wee1B | ISSPQVEEGDSR |
| KinobeadDP | YES | LLLNPGNQR |
| KinobeadDP | YES | LPQLVDMAAQIADGMAYIER |
| KinobeadDP | YES | SDVWSFGILQTELVTK |
| KinobeadDP | YES | AANILVGENLVCK |
| KinobeadDP | YES | EVLEQVER |
| KinobeadDP | YES | FQIINNTEGDWWEAR |
| KinobeadDP | YES | WTAPEAALYGR |
| KinobeadDP | YES | LIEDNEYTAR |
| KinobeadDP | YES | GSLLDFLK |
| KinobeadDP | YES | IADFGLAR |
| KinobeadDP | ZAK | CEIEATLER |
| KinobeadDP | ZAK | GLEGLQVAWLVVEK |
| KinobeadDP | ZAK | LTIPSSCPR |
| KinobeadDP | ZAK | NVVIAADGVLK |

### Example 3: Screening assay using test compounds for protein elution

This example illustrates competitive elution of proteins bound to kinobeads with non-modified test compounds (see particularly the fourth aspect of the invention). The kinobeads (as described in example 1) were contacted with mouse brains lysate, bound proteins were eluted with various test compounds and the released proteins were analysed by mass spectrometry.

### 1. Preparation of the biological sample (tissue lysate)

### 1.1 Preparation of lysates

A mouse brain lysate was prepared by mechanical disruption in lysis buffer (5 ml buffer per mouse brain) under gentle conditions that maintain the structure and function of proteins. The following steps were performed:
- Thaw the tissue quickly at room temperature or 37°C, then transfer tissue to a glass bottle containing the 1x lysis buffer (use a vial big enough to be used with Polytron PT 3100 homogenizer)
- Lyse the organ/tissue with 4x 10 sec pulses at 5000-7000 rpm at 4°C in the cold room
- Transfer the homogenate into precooled 50 ml falcon tubes
- Incubate homogenate on ice for 30 min
- Spin cells for 10 min at 6000 g at 4°C (6.000 rpm in Sorvall SLA600, precooled)
- Transfer supernatant to a UZ-polycarbonate tube (Beckmann, 355654)
- Spin supernatant for 1 h at 145.000 g at 4°C (40.000 rpm in Ti50.2, precooled)
- Save supernatant (remove and discard most of the lipid layer if possible), transfer supernatant into a glass bottle and store on ice
- Determine protein concentration by Bradford assay (BioRad). Typical protein concentrations are in the range of 5-10 mg/ml.
- Prepare aliquots in 15 to 50 ml Falcon tubes
- Freeze aliquots in liquid nitrogen and store them at -80°C

### 1.2 Preparation of lysis buffer and stock solutions

### Preparation of 100 ml 1x lysis buffer with 0.8% NP40

Combine the following solutions or reagents and add destilled water to a final volume of 100 ml: 20 ml 5x lysis buffer (see below), 100 µl 1M DTT, 5 ml 0.5 M NaF, 4 ml 20% NP40, 4 complete EDTA-free tablets (protease inhibitor cocktail, Roche Diagnostics, 1 873 580), add distilled water to 100 ml.

**Table 10: Preparation of 5x-lysis buffer**

| **Substance:** | **Stock solution** | **Final conc. in 1 x lysis buffer** | **Add for 11 5 x lysis buffer** |
|---|---|---|---|
| **Tris/HCl pH 7.5** | 1 M | 50 mM | 250 ml |
| **Glycerol** | 87% | 5% | 288 ml |
| **MgCl₂** | 1M | 1.5 mM | 7.5 ml |
| **NaCl** | 5 M | 150 mM | 150 ml |
| **Na₃VO₄** | 100 mM | 1 mM | 50 ml |

These solutions were obtained from the following suppliers:
1M Tris/HCl pH 7.5: Sigma, T-2663; 87% Glycerol: Merck, cat.no. 04091.2500; 1 M MgCl₂: Sigma, M-1028; 5 M NaCl: Sigma, S-5150.
The 5x concentrated lysis buffer was filtered through a 0.22 µm filter and stored in 40 ml aliquots at -80°C.

### Preparation of stock solutions

### Preparation of 100 mM Na₃VO₄ stock solution:

Dissolve 9,2 g Na₃VO₄ in 400 ml distilled water.
1) Adjust the pH to 10.0 using either 1 N NaOH or 1 N HCl. The starting pH of the sodium orthovanadate solution may vary with batch. At pH 10.0 the solution will be yellow.
2) Boil the solution until it turns colorless (approximately 10 min).
3) Cool to room temperature.
4) Readjust the pH to 10.0 and repeat steps 2 and 3 until solution remains colorless and the pH stabilizes at 10.0.
5) Adjust the volume to 500 ml with distilled water.
6) Freeze aliquots at -20°C. Aliquots can be stored for several months.

### Preparation of 500 mM NaF stock solution:

Dissolve 21.0 g NaF (Sigma, S7920) in 500 ml distilled water. Filter solution through 0.22 µm filter and store at 4°C.

### Preparation of 20% NP40-solution:

Weigh 40.0 g NP40 (Sigma, Igepal-CA630, catalogue No. 13021). Add distilled water up to 200 g. Mix completely and store solution at room temperature.

### Preparation of 1 M DTT solution:

Dissolve 7.7 g DTT (Biomol, catalogue No. 04010) in 50 ml distilled water. Filter solution through 0.22 µm filter and freeze 400 µl aliquots at -20°C. Aliquots can be stored for several months.

### 2. Contacting of the kinobeads with the cell lysate and elution of bound proteins by test compounds

The kinobeads were contacted with mouse brain lysate under conditions that allow the binding of the proteins in the lysate to the ligands. The binding conditions were close to physiological by choosing suitable buffer conditions preserving the function of the proteins. After removing non-captured proteins through a gentle washing procedure the bound proteins were contacted with a test compound for protein elution.

### 2.1 Preparation of DP-buffers

**Table 11: Preparation of 5x-DP buffer**

| **Substance:** | **Stock solution** | **Final conc. in 1 x lysis buffer** | **Add for 11 5 x lysis buffer** |
|---|---|---|---|
| **Tris/HCl pH 7.5** | 1 M | 50 mM | 250 ml |
| **Glycerol** | 87% | 5% | 288 ml |
| **MgCl₂** | 1 M | 1.5 mM | 7.5 ml |
| **NaCl** | 5 M | 150 mM | 150 ml |
| **Na₃VO₄** | 100 mM | 1 mM | 50 ml |

The 5x-DP buffer is filtered through 0.22 µm filter and stored in 40 ml-aliquots at -80°C.
(Note: The same buffer is also used for the preparation of total cell lysates.)
These solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

### The following 1x DP buffers were prepared

- 1x DP buffer (for bead equilibration)
- 1x DP buffer/0.4% NP40 (for bead equilibration and first wash step of beads)
- 1x DP buffer/0.2% NP40 (for second wash step of beads and for compound elution)
- 1x DP buffer/protease inhibitors (for first lysate dilution step); add one protease inhibitor tablet per 25 ml lysis buffer (EDTA-free tablet, protease inhibitor cocktail, Roche Diagnostics, 1 873 580)
- 1x DP buffer/0.4% NP40/protease inhibitors (for second lysate dilution step)

### Example for preparation of 1x DP-buffer/0.4% NP40 (100 ml)

Combine the following solutions and reagents and add distilled water up to a final volume of 100 ml: 20 ml 5x DP buffer, 5 ml 0.5 M NaF, 2 ml 20% NP40, 100 µl 1 M DTT, and add distilled water up to 100 ml. All buffers contain 1 mM DTT final concentration.

### 2.2 Washing and equilibration of Beads

The kinobeads (Example 1) were prepared for the binding reaction by washing with a suitable buffer and equilibration in the buffer.
1. Use 15 ml Falcon tubes for all washing steps.
2. Use 100 µl KinoBeads per experiment (settled bead volume): mix equal amounts (25 µl) of each bead type coupled with the following 4 ligands (coupling density of 1 µmol/ml): Bis VIII (CZC00001056), Purvalanol B (CZC00007097), PD173955 derivative (CZC00007324), and CZC00008004.
3. Wash beads two times with 3 ml 1x DP buffer and once with 3 ml 1x DP buffer/0.4% NP40. During each wash step invert tubes 3-5 times, centrifuge 2 minutes at 1200 rpm at 4°C in a Heraeus centrifuge. Supernatants are aspirated supernatants and discarded.

After the last washing step prepare a 1:1 slurry (volume/volume) with 1x DP buffer/0.4% NP40.

### 2.3 Preparation of diluted cell lysate

The mouse brain lysate was prepared for the binding reaction by dilution in a suitable buffer and clearing through a centrifugation step.
1. Use a volume of cell lysate corresponding to 50 mg protein per experiment.
2. Thaw the lysate quickly in 37°C water bath, then keep the sample on ice.
3. Dilute the lysate in the following way:
   3) dilute lysate with 1x DP buffer/protease inhibitors to reduce detergent concentration from 0.8% to 0.4% NP-40.
   4) dilute lysate further with 1x DP buffer/0.4% NP40/protease inhibitors to reach a final protein concentration of 5 mg/ml.
      (Note: The second dilution step is only required if the protein concentration of the lysate after the first dilution step is higher than 5 mg/ml).
4. Transfer diluted lysate into UZ-polycarbonate tube (Beckmann, 355654).
5. Clear diluted lysate through ultracentrifugation (20 min, 4°C, 100.000 g, TI50.2 rotor, precooled ultracentrifuge).
6. Save supernatant and keep it on ice.

### 2.4 Binding reaction and washing

The washed and equilibrated beads (section 2.2) were contaced with the diluted cell lysate (section 2.3) in order to allow binding of proteins to the ligands. Non-specifically bound proteins were removed by gentle washing in order to reduce background binding.
1. Combine diluted cleared lysate with 100 µl of washed KinoBeads in 15 ml or 50 ml Falcon tube.
2. Incubate for 2 hours at 4°C, rotate on ROTO SHAKE GENIE (Scientific Industries, Inc.) in cold room.
3. After incubation centrifuge for 3 minutes at 1200 rpm in a Heraeus centrifuge or equivalent at 4°C.
4. Remove supernatant carefully without loosing the beads.
5. Transfer the beads to a Mobicol-columns with 90 µm filter (MoBiTec, Goettingen, Cat.no: M1002-90).
6. Wash beads with 10 ml 1x DP buffer/0.4% NP-40 and 5 ml 1x DP buffer/0.2% NP-40.
7. Let washing buffer run through the column completely before proceeding with next step
8. Place column in Eppendorf tube and centrifuge them for 1 minute at 800 rpm at 4°C.

Close columns with lower lid.

### 2.5 Elution of bound proteins with test compounds

Various test compounds (see section 2.7) were used to release bound proteins follwing these steps:
1. Resuspend beads with bound proteins (section 2.4) in 1x DP buffer/0.2% NP-40 as 1:3 slurry (volume/volume).
2. Transfer 20 µl the 1:3 slurry into MoBiTech columns (equivalent to 5 µl of beads).
3. Place the column into an Eppendorf tube and centrifuge it for 15 seconds at 800 rpm at 4°C. Close columns with lower lid.
4. Add 10 µl elution buffer containing 1.0 mM of the test compound (concentration ranges of 0.1 to 1.0 mM are suitable). As a control use elution buffer containing 2% DMSO or 2% DMF dependent on the solvent used for dissolving the test compound.

Close column with upper lid.
5. Incubate for 30 minutes at 4°C in an Eppendorf incubator at 700 rpm.
6. Open column (first top, then bottom), put column back into siliconized tube (SafeSeal Microcentrifuge Tubes, cat.no 11270, Sorenson BioScience, Inc.). To harvest the eluate centrifuge 2 minutes at 2.000 rpm in table top centrifuge at room temperature. The typical volume of the eluate is approximately 15 µl.
7. Add 5 µl 4x NuPAGE SDS Sample Buffer (Invitrogen, NP0007) containing 100 mM DTT (DTT has to be added just prior to use).
8. Incubate for 30 minutes at 50°C.
9. Add 1/10 volume of 200 mg/ml iodoacetamide, incubate for 30 min at room temperature, protect from light. This reaction leads to the alkylation of cysteines for mass spectrometry.
10. Before loading samples onto the gel, centrifuge samples for 5 minutes at 15.000 rpm in order to remove precipitates.
11. For protein separation apply □ 10 µl sample to NuPAGE 4-12% Bis-Tris gel (Invitrogen, NP0335).

### 2.6 Preparation of stock solutions used in this protocol

### Preparation of a 100 mM Na₃VO₄ stock solution

1) Dissolve 9.2 g Na₃VO₄ in 400 ml distilled water.
2) Adjust the pH to 10.0 using either 1 N NaOH or 1 N HCl. The starting pH of the sodium orthovanadate solution may vary with batch. At pH 10.0 the solution will be yellow.
3) Boil the solution until it turns colorless (approximately 10 min).
4) Cool to room temperature.
5) Readjust the pH to 10.0 and repeat steps 2 and 3 until solution remains colorless and the pH stabilizes at 10.0.
6) Adjust the volume to 500 ml with distilled water.
7) Freeze aliquots at -20°C. Aliquots can be stored for several months.

### Preparation of a 500 mM NaF stock solution

Dissolve 21.0 g NaF (Sigma, S7920) in 500 ml distilled water. Filter solution through a 0.22 µm filter and store at 4°C.

### Preparation of a 20% NP40-solution

Weigh 40.0 g NP40 (Sigma, Igepal-CA630, cat.no. 13021). Add distilled water up to 200 g. Mix completely and store solution at room temperature.

### Preparation of a 1 M DTT solution

Dissolve 7.7 g DTT (Biomol, catalogue number 04010) in 50 ml distilled water. Filter solution through 0.22 µm filter and freeze 400 µl aliquots at -20°C. Aliquots can be stored for several months.

### Preparation of a Iodoacetamide stock solution (200 mg/ml)

Dissolve 2.0 g Iodoacetamide (Sigma, I-6125) in 10 ml distilled water. Filter solution through 0.22 µm filter and freeze 400 µl aliquots at -20°C. Aliquots can be stored for several months.

### 2.7 Test compounds for elution

The test compounds listed below were used for elution experiments after dilution as described below.

### Preparation of test compound stocks:

Typically all compounds are dissolved in 100 % DMSO (Fluka, cat.no 41647) at a concentration of 100 mM. Alternatively, 100 % DMF (Fluka, cat.no 40228) can be used for those compounds which cannot be dissolved in DMSO. Compounds are stored at - 20°C.

### Dilution of test compound for elution experiments:

Prepare 50 mM stock by diluting the 100 mM stock 1:1 with 100% DMSO. For elution experiments further dilute the compound 1:50 with elution buffer (= 1x DP-buffer/0,2% NP40).

**CZC1038: Bisindolylmaleimide III** (Supplier: Alexis Biochemicals; catalogue number 270-051-M005; Chemical Formula-C₂₃H₂₀N₄O₂; MW 384.4).

**CZC7097: Purvalanol B** (Supplier: Tocris Biochemicals, catalogue number 1581; chemical composition C₂₀H₂₅ClN₆O₃; MW 441.92; CAS number 212844-54-7).

**CZC00007324** (PD173955 derivative; the syntheis is described in Exanple 1.

**CZC00008004:** This is an analog of CZC00004919 (synthesis is decribed in Example 1).

**CZC00007098: SB202190** (Supplier: TOCRIS 1264 1A/46297; chemical formula C₂₀H₁₄N₃OF; MW 331.34).

**CZC00009280: Staurosporine** (Supplier: SIGMA-ALDRICH: S4400; broad range kinase inhibitor; chemical formula C₂₈H₂₆N₄O₃; MW 466.53).

**CZC00007449: SP600125** (Supplier: Merck Biosciences #420119, JNK1/2 inhibitor; chemical formula C₁₄H₈N₂O; MW 220.2).

### 3. Mass spectrometric analysis of eluted enzymes (e.g. kinases)

### Description of proteotypic peptides

Tryptic digestion of a SDS-PAGE-separated protein mixture generates for each protein numerous distinct peptide fragments with different physico-chemical properties. These peptides differ in compatibility with the mass spectrometry-based analytical platform used for protein identification (ID), here nanocapillary reversed phase-liquid chromatography electrospray ionization tandem mass spectrometry (RP-LC-MS/MS). As a result, peptide frequencies for peptides from the same source protein differ by a great degree, the most frequently observed peptides that "typically" contribute to the identification of this protein being termed "proteotypic" peptides. Thus, a "proteotypic peptide" is an experimentally well observable peptide that uniquely identifies a specific protein or protein isoform.

### Advantages of proteotypic peptides

The use of proteotypic peptides for protein identification allows rapid and focussed identification and quantitation of multiple known target proteins by focusing the protein identification process on a screening for the presence of information-rich signature peptides.

### Experimental identification of proteotypic peptides

One strategy to generate a list of proteotypic peptides is to collect peptide-identification data empirically and to search the dataset for commonly observed peptides that uniquely identify a protein.

For each IPI database protein entry with at least 10 unequivocal identifications in the CZ dataset (multipeptide IDs or manually verified single peptide IDs), peptide frequencies for contributing peptides are calculated. Only specific, best peptide-to-spectrum matches according to the database search engine Mascot™ (Matrix Science) are considered.

For definition of proteotypic peptides for a specific protein, peptides are ordered by descending peptide frequency and a cumulative peptide presence is calculated: This value gives for each peptide the ratio of identifications where this peptide or any of the peptides with a higher peptide frequency was present. Proteotypic peptides are defined using a cutoff for cumulative presence of 95%, i.e. at least 95% of identification events for this protein were based on at least one proteotypic peptide.

### 3.1 Protein digestion and sample preparation prior to mass spectrometric analysis

Proteins were concentrated, separated on 4-12% NuPAGE® Novex gels (Invitrogen, Carlsbad, CA), and stained with colloidal Coomassie blue. Gel lanes were systematically cut across the entire separation range into ≤ 48 slices (bands and interband regions) and subjected to in-gel tryptic digestion essentially as described by Shevchenko et al., 1996, Analytical Chemistry 68: 850-858. Briefly, gel plugs were destained overnight in 5mM NH₄HCO₃ in 50%EtOH, digested with for 4 hours with trypsin at 12.5 ng/µl in 5mM NH₄HCO₃. Peptides were extracted with 1% formic acid, transferred into a second 96 well plate and dried under vacuum. Dry peptides were resuspended 10 µl 0.1 % formic acid in water and 5 µl were injected into the LC-MS/MS system for protein identification.

### 3.2 Mass spectrometric data acquisition

Peptide samples were injected into a nano LC system (CapLC, Waters or Ultimate, Dionex) which was directly coupled either to a quadrupole time-of-fligth (QTOF2, QTOF Ultima, QTOF Micro, Micromass or QSTAR Pulsar, Sciex) or ion trap (LCQ Deca XP, LTQ, Thermo-Finnigan) mass spectrometer. Peptides were separated on the LC system using a gradient of aqueous and organic solvents with a typical gradient time of between 15 and 45 min. Solvent A was 5% acetonitrile in 0.1% formic acid and solvent B was 70% acetonitrile in 0.1 % formic acid.

### 3.3 Protein identification

The peptide mass and fragmentation data generated in the LC-MS/MS experiments were used to query an in-house curated version of the International Protein Index (IPI) protein sequence database (EBI) Proteins were identified by correlating the measured peptide mass and fragmentation data with the same data computed from the entries in the database using the software tool Mascot (Matrix Science; Perkins et al., 1999, Electrophoresis 20: 3551-3567). Search criteria varied depending on which mass spectrometer was used for the analysis.

### 4. Results

The method allows to establish a profile of the eluted kinases for any given test compound thereby allowing to assess the selectivity of the test compound. One limitation is that only the kinases captured on the kinobeads during the first step can be assessed, which might represent a subfraction of all kinases contained within the cell lysate.

**Table 12: Released kinases identified by mass spectrometry analysis after specific elution with test compounds (numbers represent mass spectrometry score/number of identified peptides). Kinase names are in accordance with the human kinase nomenclature (http://kinase.com) and Manning et al., 2002, Science 298, 1912-1934 as specified in supplementary material). The first four compounds in table 12 are the non-immobilized versions of the kinobead ligands 1-4 .**

| Kinase | 2% DMSO | CZC1038 | CZC7097 | CZC7324 | CZC8004 | CZC1355 | CZC7098 | CZC7479 | CZC9280 | SDS |
|---|---|---|---|---|---|---|---|---|---|---|
| AAK1 | 130/5 | 562/38 | 1036/51 | 582/35 | 718/35 | 638/27 | 198/4 | 593/32 | 777/44 | 240/32 |
| ALK2 | | | | | | | | | | 65/2 |
| AMPKa1 | | | | | 64/2 | | | | | |
| ARG | | | | 59/2 | | | | | | 452/28 |
| BRAFps | | | | 70/4 | | | | | | |
| CaMK2a | | 911/53 | 978/27 | 513/11 | 966/43 | 288/9 | | 509/11 | 1027/69 | 786/74 |
| CaMK2b | | 834/52 | 679/16 | 499/14 | 685/30 | 572/19 | | 143/5 | 1182/72 | 615/75 |
| CaMK2d | | 702/17 | 473/12 | 328/7 | 444/11 | 260/8 | | | 822/40 | 434/36 |
| CaMK2g | | 550/18 | | | 527/27 | | | | 809/25 | 379/42 |
| CaMKK2 | | 314/6 | 465/9 | 101/3 | 522/12 | 93/2 | | | 282/10 | 338/10 |
| CDK5 | 303/7 | 831/19 | 1086/41 | 462/11 | 794/27 | 856/28 | 166/4 | 504/11 | 861/43 | 604/21 |
| CDKL5 | | | 48/1 | | | | | | | |
| CK1e | | | | | | | | | | |
| CRIK | | 90/5 | | | | | | | | |
| CSK | | | 492/12 | 809/19 | | | | | 307/8 | 365/15 |
| EphA4 | | | 1702/37 | 1233/32 | 138/3 | | | | 332/11 | 727/33 |
| EphA5 | | | 330/14 | 144/3 | | | | | | 246/9 |
| EphA7 | | | | | | | | | | |
| EphB1 | | | 366/10 | | | | | | | 180/10 |
| EphB2 | | | 1334/30 | 1004/22 | 149/5 | | | | 114/4 | 437/19 |
| Erk1ps5 | | 418/14 | 749/48 | 557/77 | 386/26 | 270/13 | | | 71/2 | 239/8 |
| Erk2 | 751/25 | 754/50 | 1437/115 | 1179/235 | 810/46 | 1036/82 | 506/20 | 641/24 | 955/50 | 1023/102 |
| FAK | | | 269/6 | 322/10 | 257/8 | | | | 379/19 | 160/6 |
| FER | | 237/6 | 322/8 | 388/11 | 100/2 | | | | 818/21 | 275/11 |
| FYN | | | 268/6 | 279/6 | | | | | 314/6 | 407/17 |
| GAK | | | | 278/7 | 733/16 | | | 90/3 | 255/6 | 166/8 |
| GSK3Aps | 166/6 | 1232/60 | 357/6 | 154/3 | 635/27 | 393/21 | 406/8 | 132/4 | 868/47 | 585/77 |
| GSK3B | | 1176/54 | 1037/30 | 789/19 | 881/26 | 536/14 | 743/34 | 345/8 | 824/59 | 763/72 |
| JNK1 | | | 311/10 | 448/17 | 590/48 | 559/18 | 540/19 | 678/28 | 457/16 | 389/47 |
| JNK2 | | 287/11 | 277/7 | 702/21 | 662/18 | 405/12 | 170/5 | 429/30 | | 369/20 |
| JNK3 | | | 284/7 | 782/49 | 642/23 | 620/37 | 542/30 | 578/39 | 363/10 | 447/27 |
| LIMK1ps | | | | | 186/4 | | | | | |
| LYN | | | | | | | | | | 134/6 |
| MAP2K1 | | 208/6 | 78/2 | | | | | 74/2 | 49/2 | 90/4 |
| MARK2 | | 49/1 | | | | | | | | |
| NEK9 | | | | | 61/2 | | | | | |
| p38a | | | | 127/6 | | | 295/10 | | | 104/7 |
| p38b | | | | 60/1 | | | 80/2 | | | 95/3 |
| PAK5 | | | | | | | | | 64/1 | |
| PKCa | | | | | | | | | 64/1 | |
| PKCg | | 107/2 | | | | | | | 53/1 | |
| PYK2 | 159/4 | 291/7 | 1909/48 | 1401/42 | 1228/58 | 88/2 | | 422/10 | 1978/82 | 809/39 |
| QSK | | | | | | | | | | 153/7 |
| RSK1 | | 292/7 | 1177/32 | 626/19 | 312/9 | 435/11 | | | 1634/47 | 825/45 |
| RSK3 | | | 401/11 | 312/12 | | | | | 721/21 | 107/6 |
| SRC | | | 544/13 | 351/9 | | | | | 546/13 | 419/25 |
| TBK1 | | | | | | | | | 48/1 | |
| TRKB | | | 207/4 | | | | | | 165/4 | |
| TRKC | | | | | | | | | | |
| YESps | | | 304/7 | | 110/3 | | | | 480/12 | 236/13 |

**Table 13: Released kinases identified by mass spectrometry analysis after specific elution with test compounds (mass spectrometry score/number of identified peptides). The test compounds are selected from a library of organic small molecules (kinase scaffold compounds). Kinase names are in accordance with the human kinase nomenclature (http://kinase.com) and Manning et al., 2002, Science 298, 1912-1934 as specified in supplementary material).**

| Kinase | CZC9413 | CZC9414 | CZC9459 | CZC9482 | CZC9493 | CZC9494 | CZC9489 | CZC9488 | CZC9484 | CZC9490 |
|---|---|---|---|---|---|---|---|---|---|---|
| AAK1 | 1110/54 | 1138/69 | 1288/69 | 1459/75 | 823/35 | 777/44 | 989/48 | 1032/57 | 663/36 | 352/16 |
| ALK2 | 85/2 | | 80/2 | 100/2 | | | | 49/1 | 106/3 | |
| AMPKa1 | | | | | | | | | | |
| ARG | | | | | | | | | | |
| BRAFps | | 96/4 | | | | | | 78/2 | | |
| CaMK2a | 909/33 | 357/8 | 1294/57 | 417/25 | 1023/49 | 1002/32 | 1430/69 | 1307/78 | 1187/83 | 71/2 |
| CaMK2b | 1128/71 | 151/5 | 1315/99 | 626/18 | 1077/77 | 806/21 | 939/67 | 1251/72 | 969/65 | |
| CaMK2d | 736/21 | | 760/42 | 414/10 | 752/24 | 376/9 | 712/39 | 720/35 | 716/24 | |
| CaMK2g | 970/25 | | 812/32 | | 709/27 | | 741/17 | 929/26 | 620/26 | |
| CaMKK2 | 242/8 | | 178/4 | 287/10 | 225/7 | | 140/4 | 187/6 | 237/6 | |
| CDK5 | 677/33 | 513/12 | 525/14 | 773/20 | 906/44 | 580/13 | 838/38 | 907/24 | 951/30 | 204/5 |
| CDKL5 | | | | | | | | | | |
| CK1e | | | | | | | | 80/2 | | |
| CRIK | | | 50/1 | | | | | | | |
| CSK | | | | | 73/2 | 66/2 | 121/2 | | 73/2 | |
| EphA4 | | | | 123/4 | | | | | | |
| EphA5 | | | | | | | | | | |
| EphA7 | | 88/2 | | | | | | | | |
| EphB1 | | | | | | | | | | |
| EphB2 | | | 74/2 | | | | | | 247/7 | |
| Erk1ps5 | 121/4 | 144/5 | 282/8 | 469/15 | 176/5 | 333/10 | 89/3 | 597/19 | 513/14 | |
| Erk2 | 913/36 | 852/37 | | 837/51 | 676/40 | 837/44 | 822/24 | 896/30 | 994/51 | 759/28 |
| FAK | | | | 302/8 | | 81/2 | 193/6 | 153/6 | | |
| FER | | | | 118/3 | | | 154/6 | 344/9 | 149/4 | |
| FYN | | | | | | | | 53/1 | | |
| GAK | 234/6 | 663/20 | 388/10 | 63/1 | 187/5 | 732/21 | 161/4 | 530/14 | 201/6 | |
| GSK3Aps | 586/18 | 891/23 | 335/7 | 230/5 | | 201/5 | 630/14 | 745/15 | 1030/55 | 78/2 |
| GSK3B | 663/32 | 471/23 | 294/14 | 143/5 | 282/8 | 257/7 | 343/8 | 567/29 | 908/60 | |
| JNK1 | | | | 595/36 | | | 553/17 | 964/34 | 703/23 | |
| JNK2 | | 394/23 | | 469/15 | 84/3 | | 523/17 | 562/30 | 494/33 | |
| JNK3 | 371/10 | 555/33 | 610/30 | 662/44 | 346/10 | 164/5 | 635/37 | 968/45 | 789/47 | |
| LIMK1ps | | | | | | | 46/1 | | | |
| LYN | | | | | | | | | | |
| MAP2K1 | 95/4 | | 253/8 | 93/3 | 234/8 | 152/5 | 105/4 | 481/12 | 338/10 | 48/1 |
| MARK2 | | | | | | | | | | |
| NEK9 | 122/3 | | | | | | | 46/1 | | |
| p38a | | | | | | | | | | |
| p38b | | | | | | | | | | |
| PAK5 | | | | | | | | | | |
| PKCa | | | | | | | | | | |
| PKCg | | | | | | 210/6 | | 145/4 | 167/5 | |
| PYK2 | 133/3 | 292/7 | 430/12 | 833/21 | 219/6 | 47/1 | 1712/42 | 1769/51 | 703/17 | 51/1 |
| QSK | | | | | | | | | | |
| RSK1 | 445/11 | 100/3 | | 193/4 | 207/4 | 511/12 | 466/14 | 1471/38 | 634/17 | |
| RSK3 | 272/8 | | 94/2 | 211/5 | | 354/11 | | 437/14 | 301/10 | |
| SRC | | | | | | | | | 135/3 | |
| TBK1 | | | | | | | | | | |
| TRKB | | | | | 139/4 | | | 111/3 | | |
| TRKC | | | | 97/3 | | | | | | |
| YESps | | | | | | | | | | |

### Example 4: Comparison of co-immobilized ligands versus separately coupled ligands

The purpose of this experiment was to assess whether a mixture of beads containing one type of ligand (separatly coupled ligands) yields similar results in terms of identified kinases compared to co-immobilized ligands (simultaneous coupling).

The result shows that there is a wide overlap of identified kinases in both experiments demonstrating that both approaches are feasible.

Two ligands were coupled either individually or simultaneously to Sepharose beads and then used for pulldown experiments using HeLa cell lysates (ligand 1: BisVIII; ligand 2: CZC00008004; details as in Example 1: Preparation of kinobeads). Coupling of the compounds individually was performed as detailed in example 1. Simultaneous coupling of the two ligands was also performed as in Example 1 except that the compounds were coupled onto the same beads at a concentration of 0.5 µmol/mL instead of the standard 1 µmol/mL beads. Coupling success of the individually or simultaneously immobilized compounds was controlled via HPLC analysis. The beads were washed and stored as described in example 1.

The preparation of HeLa cell lysates, bead washing, contacting of the beads with lysate, washing and analsyis of released proteins by mass spectrometrydrug were preformed as described in Example 2 (Signalokinome experiment).

**Table 14: Kinases identified by mass spectrometry analysis. Comparison of the experiment with two simultaneously coupled ligands (left part) versus individually coupled compounds (mixed beads; right part). Kinase names are in accordance with the human kinase nomenclature (http://kinase.com) and Manning et al., 2002, Science 298, 1912-1934 as specified in supplementary material).**

| **Simultaneous coupling (Bis VIII and CZC8004 pre-mixed)** | | | | | **Beads mixed post-coupling (Bis VIII and CZC8004)** | | | | |
|---|---|---|---|---|---|---|---|---|---|
| **Identification** | **Kinase** | **MS Score** | **Number of peptides** | **also in post mix** | **Identification** | **Kinase** | **MS Score** | **Number of peptides** | **also in pre mix** |
| 1 | TBK1 | 1531 | 63 | X | 1 | TBK1 | 1365 | 57 | X |
| 2 | GSK3B | 932 | 52 | X | 2 | NEK9 | 1113 | 36 | X |
| 3 | AurA | 895 | 40 | X | 3 | TNK1 | 921 | 44 | X |
| 4 | TYK2 | 841 | 29 | X | 4 | TYK2 | 917 | 34 | X |
| 5 | TNK1 | 771 | 46 | X | 5 | AurA | 910 | 46 | X |
| 6 | CDK2 | 741 | 34 | X | 6 | GSK3A | 814 | 47 | X |
| 7 | NEK9 | 736 | 22 | X | 7 | CaMK2g | 781 | 38 | X |
| 8 | GSK3A | 707 | 43 | X | 8 | GSK3B | 745 | 33 | X |
| 9 | JNK2 | 654 | 24 | X | 9 | JAK1 | 731 | 27 | X |
| 10 | JNK1 | 548 | 23 | X | 10 | CaMK2d | 641 | 26 | X |
| 11 | CaMK2g | 494 | 24 | X | 11 | AurB | 631 | 28 | X |
| 12 | CaMK2d | 456 | 21 | X | 12 | DNAPK | 610 | 24 | X |
| 13 | AAK1 | 450 | 17 | X | 13 | JNK2 | 578 | 23 | X |
| 14 | AurB | 424 | 15 | X | 14 | JNK1 | 499 | 21 | X |
| 15 | JAK1 | 187 | 6 | X | 15 | CDK2 | 485 | 19 | X |
| 16 | CDK9 | 160 | 6 | 0 | 16 | FER | 423 | 17 | X |
| 17 | YES | 108 | 5 | X | 17 | AAK1 | 368 | 17 | X |
| 18 | FER | 106 | 3 | X | 18 | TEC | 159 | 7 | 0 |
| 19 | MPSK1 | 80 | 3 | 0 | 19 | BIKE | 124 | 4 | 0 |
| 20 | DNAPK | 72 | 4 | X | 20 | YES | 108 | 6 | X |
| 21 | ULK3 | 64 | 1 | 0 | 21 | FRAP | 59 | 3 | 0 |

**Table 15: Kinases and sequences of proteotypic peptides identified in the simultaneous coupling experiment**

| **KINASE NAME** | **PROTEOTYPIC PEPTIDE** |
|---|---|
| AAK1 | APEMVNLYSGK |
| AURKB | IYLILEYAPR |
| AURKB | LPLAQVSAHPWVR |
| AURKB | SNVQPTAAPGQK |
| CAMK2D | DLKPENLLLASK |
| CAMK2D | FTDEYQLFEELGK |
| CAMK2D | GAILTTMLATR |
| CAMK2D | IPTGQEYAAK |
| CAMK2G | LTQYIDGQGRPR |
| CDK2 | ALFPGDSEIDQLFR |
| CDK2 | FMDASALTGIPLPLIK |
| CDK9 | IGQGTFGEVFK |
| CDK9 | LLVLDPAQR |
| CDK9 | NPATTNQTEFER |
| GSK3A | SQEVAYTDIK |
| GSK3A | VIGNGSFGVVYQAR |
| GSK3A | VTTVVATLGQGPER |
| GSK3A | YFFYSSGEK |
| GSK3B | DIKPQNLLLDPDTAVLK |
| GSK3B | LYMYQLFR |
| GSK3B | VIGNGSFGVVYQAK |
| JAK1 | LIMEFLPSGSLK |
| JAK1 | LSDPGIPITVLSR |
| MAPK8 | APEVILGMGYK |
| MAPK9 | ILDFGLAR |
| MAPK9 | NIISLLNVFTPQK |
| MAPK9 | VIEQLGTPSAEFMK |
| NEK9 | LGINLLGGPLGGK |
| NEK9 | LNPAVTCAGK |
| NEK9 | LQQENLQIFTQLQK |
| NEK9 | SSTVTEAPIAVVTSR |
| NEK9 | VLACGLNEFNK |
| PRKDC | INQVFHGSCITEGNELTK |
| PRKDC | NELEIPGQYDGR |
| PRKDC | QLFSSLFSGILK |
| PRKDC | YPEETLSLMTK |
| STK16 | APELFSVQSHCVIDER |
| STK16 | GTLWNEIER |
| STK6 | SKQPLPSAPENNPEEELASK |
| STK6 | VEFTFPDFVTEGAR |
| TBK1 | IASTLLLYQELMR |
| TBK1 | LFAIEEETTTR |
| TBK1 | TTEENPIFVVSR |
| TBK1 | VIGEDGQSVYK |
| TNK1 | AAALSGGLLSDPELQR |
| TNK1 | VADFGLVRPLGGAR |
| TYK2 | LGLAEGTSPFIK |
| TYK2 | LSDPGVGLGALSR |
| TYK2 | MVVAQQLASALSYLENK |
| TYK2 | SLQLVMEYVPLGSLR |
| TYK2 | SQAPDGMQSLR |
| ULK3 | ASVENLLTEIEILK |
| YES1 | AANILVGENLVCK |
| YES1 | EVLEQVER |

**Table 16: Kinases and sequences of proteotypic peptides identified in the mix experiment (beads mixed post coupling)**

| **KINASE NAME** | **PROTEOTYPIC PEPTIDE** |
|---|---|
| AAK1 | ADIWALGCLLYK |
| AURKB | HFTIDDFEIGRPLGK |
| AURKB | IYLILEYAPR |
| AURKB | LPLAQVSAHPWVR |
| AURKB | SNVQPTAAPGQK |
| BMP2K | ADIWALGCLLYK |
| BMP2K | ITDTIGPTETSIAPR |
| CAMK2D | AGAYDFPSPEWDTVTPEAK |
| CAMK2D | FTDEYQLFEELGK |
| CAMK2D | FYFENALSK |
| CAMK2D | GAILTTMLATR |
| CAMK2G | AGAYDFPSPEWDTVTPEAK |
| CAMK2G | DLKPENLLLASK |
| CAMK2G | FTDDYQLFEELGK |
| CAMK2G | LTQYIDGQGRPR |
| CAMK2G | NLINQMLTINPAK |
| CDK2 | ALFPGDSEIDQLFR |
| CDK2 | FMDASALTGIPLPLIK |
| FER | HSIAGIIR |
| FER | THAEDLNSGPLHR |
| FRAP1 | GPTPAILESLISINNK |
| GSK3A | SQEVAYTDIK |
| GSK3A | VIGNGSFGVVYQAR |
| GSK3A | VTTVVATLGQGPER |
| GSK3A | YFFYSSGEK |
| GSK3B | DIKPQNLLLDPDTAVLK |
| GSK3B | LLEYTPTAR |
| GSK3B | LYMYQLFR |
| GSK3B | VIGNGSFGVVYQAK |
| GSK3B | YFFYSSGEK |
| JAK1 | LIMEFLPSGSLK |
| JAK1 | LSDPGIPITVLSR |
| MAPK8 | APEVILGMGYK |
| MAPK8 | NIIGLLNVFTPQK |
| MAPK9 | APEVILGMGYK |
| MAPK9 | ILDFGLAR |
| MAPK9 | NIISLLNVFTPQK |
| MAPK9 | VIEQLGTPSAEFMK |
| NEK9 | AGGGAAEQEELHYIPIR |
| NEK9 | LNPAVTCAGK |
| NEK9 | LQQENLQIFTQLQK |
| NEK9 | SSTVTEAPIAVVTSR |
| NEK9 | VLACGLNEFNK |
| PRKDC | AALSALESFLK |
| PRKDC | DFGLLVFVR |
| PRKDC | DILPCLDGYLK |
| PRKDC | DLLLNTMSQEEK |
| PRKDC | DQNILLGTTYR |
| PRKDC | FMNAVFFLLPK |
| PRKDC | FVPLLPGNR |
| PRKDC | GLSSLLCNFTK |
| PRKDC | HSSLITPLQAVAQR |
| PRKDC | INQVFHGSCITEGNELTK |
| PRKDC | LAGANPAVITCDELLLGHEK |
| PRKDC | LATTILQHWK |
| PRKDC | LSDFNDITNMLLLK |
| PRKDC | LYSLALHPNAFK |
| PRKDC | NELEIPGQYDGR |
| PRKDC | QCLPSLDLSCK |
| PRKDC | SDPGLLTNTMDVFVK |
| PRKDC | SIGEYDVLR |
| PRKDC | SLGPPQGEEDSVPR |
| PRKDC | VTELALTASDR |
| STK6 | QWALEDFEIGRPLGK |
| STK6 | SKQPLPSAPENNPEEELASK |
| STK6 | VEFTFPDFVTEGAR |
| TBK1 | IASTLLLYQELMR |
| TBK1 | LFAIEEETTTR |
| TBK1 | TTEENPIFVVSR |
| TBK1 | VIGEDGQSVYK |
| TEC | ELGSGLFGVVR |
| TEC | GQEYLILEK |
| TEC | HAFGSIPEIIEYHK |
| TNK1 | AAALSGGLLSDPELQR |
| TNK1 | ANLWDAPPAR |
| TNK1 | MLPEAGSLWLLK |
| TNK1 | VADFGLVRPLGGAR |
| TYK2 | AAALSFVSLVDGYFR |
| TYK2 | HGIPLEEVAK |
| TYK2 | LGLAEGTSPFIK |
| TYK2 | LSDPGVGLGALSR |
| TYK2 | MVVAQQLASALSYLENK |
| TYK2 | SLQLVMEYVPLGSLR |
| TYK2 | SQAPDGMQSLR |
| YES1 | EVLEQVER |

### Example 5: Synthesis of kinobead ligand 5 (indol ligand 91)

### Synthesis indol ligand 91 : 5-[5-Fluoro-2-oxo-1,2-dihydro-indol-(3Z)-ylidenemethyl]-2,4-dimethyl-1H-pyrrole-3-carboxylic acid (3-amino-propyl)-amide

### Step 1: 5-Fluoro-1,3-dihydro-indol-2-one

A solution of 5-Fluoroisatin (1g) in Hydrazine hydrate (55%, 10ml) was heated at 110°C for 30minutes. Once the suspension has gone into solution, the reaction was heated at 110°C for 4 hours, then cooled at 0°C. The precipitate was filtered and washed with water. The solid was suspended in water (10ml:), the pH was lowered to pH2 by addition of HCl conc, and the solution stirred at room temperature for 5 hours. The precipitate was collected, the solid washed with water (2x15ml) and dried in the vacuum oven at 40°C (0.26g,30%). ¹H NMR (400 MHz, DMSO-*d₆*) δ 10.2 (s, 1H); 6.8-7.0 (dd, 2H); 6.6 (m, 1H); 3.2 (s, 2H);. LCMS: method D, RT=1.736min, [MH⁺=152].

### Step 2: {3-[(5-Formyl-2,4-dimethyl-1H-pyrrole-3-carbonyl)-amino]-propyl}-carbamic acid tert-butyl ester

To a solution of 5-formyl-2,4-dimethyl-1H-pyrazol-3-carboxylic acid (0.300g, 1.79mmol), N-(3-Dimethylaminopropyl)-N'-ethylcarbodiimide (0.516g, 2.69mmol), 1-Hydroxybenzotriazole hydrate (0.364g, 2.69mmol), triethylamine (0.502ml, 3.56mmol) in dimethylformamide (3ml) was added N-Boc-1,3-diaminopropane (0.375ml, 2.15mmol). The solution was stirred at room temperature for 15 hours. A mixture of brine (1.5ml), water (1.5ml) and saturated aqueous sodium bicarbonate (1.5ml) was added and the pH of the solution adjusted to 12 by addition of 10N sodium hydroxide. The solution was extracted 3 times with a mixture of dichloromethane: Methanol (9:1). The organic layer was dried with anhydrous magnesium sulfate. The solvent was removed and the residue purified by flash chromatography (Hexane : Ethyl acetate(50 to 100%)) to yield the desired compound as a yellow solid (0.30g, 52%). ¹H NMR (400 MHz, DMSO-*d₆*) δ 11.90 (s, 1H); 9.50 (s, 1H); 7.50 (m, 1H); 6.90 (m, 1H); 3.20 (q, 2H); 3.00 (q, 2H); 2.30 (s, 3H)); 2.20 (s, 3H) ); 1.60 (m, 2H) ); 1.40 (s, 9H). LCMS: method D, RT=2.103min, [M+Na⁺=346], and [M-Boc+Na⁺=246].

### Step 3: [3-({5-[5-Fluoro-2-oxo-1,2-dihydro-indol-(3Z)-ylidenemethyl]-2,4-dimethyl-1H-pyrrole-3-carbonyl}-amino)-propyl]-carbamic acid tert-butyl ester

A solution of {3-[(5-Formyl-2,4-dimethyl-1H-pyrrole-3-carbonyl)-amino]-propyl}-carbamic acid tert-butyl ester (0.200g, 0.62mmol) and 5-Fluoro-1,3-dihydro-indol-2-one (0.011g, 0.62mmol), pyrrolidine (0.003ml) in ethanol (2ml) was heated at 78°C for 3 hours. The reaction was cooled to 0°C and the resulting precipitate filtered, washed with cold ethanol. The product was suspended in ethanol (4ml) and stirred at 72°C for 30minutes. The reaction was filtered, the precipitate dried in a vacuum oven at 40°C to yield the desired compound as a solid (0.265g, 94%). ¹H NMR (400 MHz, DMSO-*d₆*) δ 13.8 (s, 1H); 11.00 (s, 1H); 7.80 (m, 2H); 7.70 (m, 1H); 7.0 (m, 1H); 6.9 (m, 2H); 3.30 (q, 2H); 3.10 (q, 2H); 2.50 (dd, 6H) 1.60 (m, 2H) ); 1.40 (s, 9H). LCMS: method D, RT=2.86min, [M+Na⁺=479], and [M-Boc+Na⁺=379].

### Step 4: 5-[5-Fluoro-2-oxo-1,2-dihydro-indol-(3Z)-ylidenemethyl]-2,4-dimethyl-1H-pyrrole-3-carboxylic acid (3-amino-propyl)-amide

[3-({5-[5-Fluoro-2-oxo-1,2-dihydro-indol-(3Z)-ylidenemethyl]-2,4-dimethyl-1H-pyrrole-3-carbonyl}-amino)-propyl]-carbamic acid tert-butyl ester was suspended in methanol. 2ml of HCl (4N) in dioxane was added and the reaction stirred at room temperature overnight. The solvent was removed to yield the desired compound (0.098g, 91 %). ¹H NMR (400 MHz, DMSO-*d₆*) δ 13.8 (s, 1H); 11.00 (s, 1H); 7.90-7.70 (m, 5H); 6.9 (m, 2H); 3.30 (q, 2H); 2.80 (q, 2H); 2.50 (dd, 6H) 1.80 (m, 2H). LCMS: inconclusive due to fluorescence.

All reactions were carried out under inert atmosphere. NMR spectra were obtained on a Bruker dpx400. LCMS was carried out on an Agilent 1100 using a zorbax SBC-18, 4.6mmx150mm-5µ column or a Small column: ZORBAX^{®} SB-C18, 4.6 x 75 mm, 3.5 microns ("short column"). Column flow was 1ml/min and solvents used were water and acetonitrile (0.1%TFA) with an injection volume of 10ul. Wavelengths were 254 and 210nm. Methods are described below.

**Table 17: Analytical methods**

| **Method** | **Easy Access Method Name** | **ChemStation Method Name** | **Flow Rate** | **Solvent** | **Run Time** |
|---|---|---|---|---|---|
| A | Analytical positive 7mn | ANL_POS7.M | 1ml/min | 0-2.5min 5-95% MeCN | 7 min |
| | | | | 2.5-6min 95% MeCN | |
| B | Analytical positive Ion | ANAL_POS.M | 1ml/min | 0-11min 5-95% MeCN | 15 min |
| | | | | 11-13min 95% MeCN | |
| C | Loop injection, Positive | | 1ml/min | 95% MeCN | 1min |
| D | Analytical positive Ion | Short column ANL Positive | 1ml/mn | 0-4.5min 30-95% MeCN | 5min |
| E | Analytical High pH | Analytical High pH | 3ml/min | 0 to 8min 5-95% MeCN | 10min |
| | | | | 8 to 9min 95% MeCN | |

**Table 18: Abbreviations used in chemistry protocols**

| | |
|---|---|
| aq | aqueous |
| D | doublet |
| DMSO | dimethyl sulfoxide |
| G | gram |
| HCl | Hydrochloric acid |
| HPLC | high pressure liquid chromatography |
| LCMS | liquid chromatography - mass spectrometry |
| M | multiplet |
| mins | minute |
| mmol | millimole |
| N | Normal |
| NMR | nuclear magnetic resonance |
| Q | quartet |
| RT | retention time |
| S | singlet |
| sat | saturated |
| T | triplet |

### Example 6: Synthesis of kinobead ligand 6 (quinazoline ligand 32)

### Synthesis of quinazoline ligand 32: 7-(4-amino)-butyloxy-4-(2-fluoro-4-bromo-phenyl)-amino-6-methoxyquinazoline

### Step 1: 7-benzyloxy-4-(2-fluoro-4-bromo-phenyl)-amino-6-methoxyquinazoline

To a solution of 7-benzyloxy-4-chloro-6-methoxyquinazoline (0.250g, 0.83mmol) and 4-bromo-2-fluoroaniline (190mg, 1mmol) in isopropanol (10ml) was added hydrochloric acid (4M in dioxane, 0.230ml, 0.92mmol). The mixture was stirred at 90°C for 2 hours. The reaction mixture was allowed to cool down to room temperature. The solid was filtered off, washed with cold isopropanol and ether and finally dried overnight at 50°C, affording the title compound (0.297g - 79%).¹H NMR (400 MHz, CD₃OD-*d₄*) δ 8.65 (s, 1H); 7.96(s, 1H); 7.55(m, 5H); 7.40(t, 3H); 7.30 (s, 1H); 5.37 (s, 2H); 4.89 (s, 1H); 4.08 (s, 1H). LCMS: method C, [M⁺=454].

### Step 2: 7-hydroxy-4-(2-fluoro-4-bromo-phenyl)-amino-6-methoxyquinazoline

7-benzyloxy-4-(2-fluoro-4-bromo-phenyl)-amino-6-methoxyquinazoline (297mg, 0.654mmol) was dissolved in trifluoroacetic acid (5ml) and the solution was stirred at reflux for one hour. The reaction mixture was allowed to cool down to room temperature and poured onto ice. The solid was filtered off and taken up in methanol. The solution was basified using aqueous ammonia (to pH11) and reduced in vacuo. The solid was collected by filtration, washed with cold water and ether and finally dried overnight under vacuum at 50°C, affording the title compound. (0.165g - 69%).¹H NMR (400 MHz, CD₃OD-*d₄*) δ 8.55 (s, 1H); 7.89(s, 1H); 7.52(m, 3H); 7.13(s, 1H); 4.08 (s, 3H). LCMS: method C, [M⁺=364].

### Step 3: 7-(4-amino-phthalimide)-butyloxy-4-(2-fluoro-4-bromo-phenyl)-amino-6-methoxyquinazo line

The N(-4-bromobutyl)-phthalimide (0.355g, 0.1.187mmol) was added in one portion to a mixture of 7-hydroxy-4-(2-fluoro-4-bromo-phenyl)-amino-6-methoxyquinazoline (360mg, 0.989mmol) and potassium carbonate (410mg, 2.967mmol) in dimethylformamide (7ml). The reaction mixture was stirred at 60°C for 2 hours and then allowed to cool down to room temperature. Water (10ml) was added and the precipitate was filtered off. The solid was washed with cold water and methanol and finally dried overnight at 50°C under vacuum, affording the title compound (0.317mg - 57%). ¹H NMR (400 MHz, CD₃OD-*d₄*) δ 9.49 (s, 1H); 8.29(s, 1H); 7.82(m, 4H); 7.71(s, 1H); 7.61(m, 1H); 7.47(t, 1H, J=8.3Hz); 7.413(m, 1H); 7.11(s, 1H); 4.09(m, 2H); 3.87(s, 3H); 3.62(m, 2H); 1.75(broad s, 4H). LCMS: method B, RT=9.20 min, [MH⁺=410].

### Step 4: 7-(4-amino)-butyloxy-4-(2-fluoro-4-bromo-phenyl)-amino-6-methoxyquinazo line

A suspension of 7-(4-amino-phthalimide)-butyloxy-4-(2-fluoro-4-bromo-phenyl)-amino-6-methoxyquinazoline (150mg, 0.265mmol) in dimethylformamide (5ml) was treated with hydrazine monohydrate. The reaction mixture was stirred at room temperature over 2 days (solubilisation occured after few minutes and total consumption of starting material observed) and then reduced in vacuo. The thick yellow oil was purified using the "catch and release" method (Isolute SCX-2 cartridge with optimised realeasing method) affording the title compound (63mg, 51%).¹H NMR (400 MHz, CDCl₃-*d₆*) δ 8.68(s, 1H); 8.48(t, 1H, J=8.5Hz); 7.36(m, 1H); 7.33(m, 1H); 7.32(broad s, 1H); 7.25(s, 1H); 7.00(s, 1H); 4.19(m, 2H); 4.02(s, 3H); 2.80(m, 2H); 1.98(m, 2H); 1.67(m, 2H); 1.49(broad s, 2H). HPLC: method D, RT=3.52 min.

All reactions were carried out under inert atmosphere. NMR spectra were obtained on a Bruker dpx400. LCMS was carried out on an Agilent 1100 using a zorbax SBC-18, 4.6mmx150mm-5µ column or a Small column: ZORBAX^{®} SB-C18, 4.6 x 75 mm, 3.5 microns ("short column"). Column flow was 1ml/min and solvents used were water and acetonitrile (0.1%TFA) with an injection volume of 10ul. Wavelengths were 254 and 210nm. Methods are described below.

**Table 19: Analytical methods**

| **Method** | **Easy Access Method Name** | **ChemStation Method Name** | **Flow Rate** | **Solvent** | **Run Time** |
|---|---|---|---|---|---|
| A | Analytical positive 7mn | ANL_POS7.M | 1ml/min | 0-2.5min 5-95% MeCN | 7 min |
| | | | | 2.5-6min 95% MeCN | |
| B | Analytical positive Ion | ANAL_POS.M | 1ml/min | 0-11min 5-95% MeCN | 15 min |
| | | | | 11-13min 95% MeCN | |
| C | Loop injection, Positive | | 1ml/min | 95% MeCN | 1min |
| D | Analytical positive Ion | Short column ANL Positive | 1ml/mn | 0-4.5min 30-95% MeCN | 5min |
| E | Analytical High pH | Analytical High pH | 3ml/min | 0 to 8min 5-95% MeCN | 10min |
| | | | | 8 to 9min 95% MeCN | |

**Table 20: Abbreviations used in chemistry protocols**

| | |
|---|---|
| aq | aqueous |
| D | doublet |
| DMSO | dimethyl sulfoxide |
| G | gram |
| HCl | Hydrochloric acid |
| HPLC | high pressure liquid chromatography |
| LCMS | liquid chromatography - mass spectrometry |
| M | multiplet |
| mins | minute |
| mmol | millimole |
| N | Normal |
| NMR | nuclear magnetic resonance |
| Q | quartet |
| RT | retention time |
| S | singlet |
| sat | saturated |
| T | triplet |

### Example 7: Synthesis of kinobead ligand 7 (modified Staurosporine)

Kinobead ligand 7 based on Staurosporine was synthesized according to the following protocol.

### Step 1: Modification of Staurosporine with diglycolic acid anhydride

Dissolve Staurosporine (typically 10 mg, IRIS-Biotech, Germany) in 1 ml waterfree DMF, add 5 µL of TEA, cool to 0 °C. From this solution, 5 µl are taken and mixed with 50 µl of ACN for determination of the relative starting amount using a LC-MS system (AGILENT, Germany). For the LC-MS analysis 5 µl of the reaction mixture is diluted into 50 µl 100 % ACN. After thorough mixing 5 µl are applied to the HPLC analysis using an autosampler. Separation is carried out at a flow rate of 450 µl/min with 0.1 % formic acid in water as solvent A and 0.1 % formic acid in 100 % ACN as solvent B using a 3 x 50 mm C18 column (ZORBAX-Extended C18, AGILENT, Germany). A gradient from 10 % A to 95 % B in 75 minutes is used. UV absorbance is observed at 254 nm, the molecular mass of the compound is determined online by a single stage quadrupol MS system (MSD, AGILENT, Germany). The recorded data are checked manually. This first analysis serves as the standard for the calculation of the yields of reaction products. The peak area of the UV signal is set to 100 % as starting amount, based on the pure Staurosporine.

To the ice cold Staurosporine solution a 10fold excess of diglycolic acid anhydride (Merck Germany) in DMF is added. The reaction with close to 100 % yield is finished within 10 minutes, check by HPLC by mixing 5 µl of the reaction mixture with 50 µl ACN and analyse by LC-MS as descibed above. The molecular mass shifts from 467.5 Da (unmodified Staurosporine) to 545.6 Da (modified Staurosporine) for the singly charged molecule. If the reaction was not complete another tenfold excess of the diglycolic acid anhydride is added and the mixture kept for another 30 minutes at room temperature. The reaction mixture is analysed by LC-MS as described above..

After the reaction is completed, no unmodified Staurosporine can be detected. 5 mL water is added to the reaction mixture, first to quench the acid anhydride, secondly to dilute for solid phase extraction. Prepare a 500 mg C18 solid phase extraction cartridge (Phenomenex, Germany) by activation with 10 ml methanol and equilibration with 0.1 % TFA in water. The solvents are drawn through the cartridge with a flow rate of approximately 2 to 3 ml/min by using a membrane vacuum pump. The aqueous reaction mixture is applied to the cartridge and drawn through it with the same flow rate as above. After the solution has completely passed through the cartridge and the modified staurosporine is bound to the C18 material, it is washed first with 5 % ACN 0.1 % TFA, then with 10 % ACN, and finally with 20 % ACN, all with 0.1 % TFA in water. The modified Staurosporine is then eluted with 70 % ACN, 0.1 % TFA in water, followed by 80 % ACN, 0.1 % TFA in water. The eluates are combined and dried in vacuum.

### Step 2: Coupling of the modified Staurosporine to the solid-phase bound diamine using PyBroP-chemistry

Dissolve the modified Staurosporine in waterfree DMF and add to pre-swollen Bis-(aminoethyl)ethylene glycol-trityl resin in DMF (IRIS Biotech, Germany). The solid phase bound diamine is added in 2 to 3 fold excess. To the slurry add 10 µl of diisopropyl ethylamine (DIEA, FLUKA, Germany) and a five fold excess of PyBroP over the modified Staurosporine. The reaction is carried out for 16 hours at room temperature under permanent mixing over an end-over-end mixer. Check the supernatant for unbound modified Staurosporine by HPLC using the LC-MS system as described above, This step is not quantitative, only the disappearance of unbound modified Staurosporine is measured. After the reaction is completed (no unbound modified Staurosporine can be detetced anymore) the resin washed with 10 volumes of waterfree DMF and two times with 10 volumes of DCM.

### Step 3: Cleavage reaction

The resin is then resuspended in 5 volumes DCM, cooled to 0 °C and 1 ml TFA is added. The former light yellow resin should now turn to dark red indicating the reaction. The cleavage is carried out for 20 minutes at 0 °C and 20 minutes at room temperature. The solution is collected and the resin washed two times with 10 volumes of DCM. All eluates are combined and dried using a rotary evaporator. The remaining oily film is dissolved in 0.5 ml DMF and 5 ml of water is added. The modified Staurosporine is purified by solid phase extraction as described above and dried under vacuum. Yield is checked by HPLC with UV absorbance at 254 nm against the original unmodified staurosporine solution as described above, relative to the solution of the unmodified Staurosporine after dissolving the reaction product in 1 ml DMF. From this solution 5 µl are mixed with 50 µl of ACN and 5 µl are analysed by LC-MS. The molecular mass of the expected product is 727.8 Da. The peak area at 254 nm is related to the peak area of the unmodified Staurosporine as 100 % analysed as described.

The modified staurosporine is used for coupling to NHS-activated sepharose as usual via its amino group. Before coupling, 1 ml of beads is washed three times with 12 ml waterfree DMSO and then resuspended with 1 mL waterfree DMSO. To this suspension 20 µl of TEA is added and an equivalent of 1 µmole of the modified Staurosporine in DMF. Directly after adding and well mixing and short centrifugation for 1 minute at 1200 rpm, 20 µl of the supernatant is taken and 5 µl is analysed by LC-MS. After 16 hours under continuous mixing on a rotary mixer, the suspension is centrifuged again and 20 µl are taken to determine the remaining unbound modified Staurosporine by analysis of 5 µl by LC-MS as described above. Usually 100 % of the modified Staurosporine is bound. The beads are washed afterwards three times with 12 ml DMSO, resuspended again with 1 ml DMSO and 50 µl of ethanolamine (MERCK, Germany) is added to block unreacted NHS-activated groups. The reaction is carried out for 16 h under continuous mixing. After the blocking reaction, the beads are washed three times with 12 ml iso-propanole (MERCK, Germany) and stored as 1:1 slurry at 4 °C until use.

The following reagents were used:
DMF: N,N-Dimethylformamide (FLUKA, 40228);
TEA: Triethylamine (SIGMA-Aldrich, T0886);
ACN: Acetonitrile for HPLC (Merck, 1.00030);
TFA: Trifluoroacetic acid (FLuKA, 09653);
PyBroP: Bromo-tris-pyrrolidino-phosphonium hexafluorophosphate (Novabiochem, 01-62-0017);
DCM: Dichlormethane (FLUKA, 66749);
DMSO: Dimethylsulfoxide (FLUKA, 41648).

### Example 8: In-lysate competition binding and Quantitative protein affinity profile (PAP)

This example illustrates competition binding in cell lysates (see particularly the third aspect of the invention). A test compound, Bisindolylmaleimide VIII (Bis VIII, a well known kinase inhibitor; Davies et al., 2000. Specificity and mechanism of action of some commonly used protein kinase inhibitors. Biochemical Journal 351(Pt 1): 95-105) was added to a cell lysate thereby allowing the test compound to bind to the target proteins in the lysate. Then the lysate was contacted with the kinobeads affinity matrix to capture remaining free target proteins. The proteins bound to the kinobeads matrix were then eluted with detergent-containing buffer, separated on a SDS-polyacryamide gel and analyzed by immunodetection (Western blots) or mass spectrometry detection.

For Western blot analysis proteins bound to the affinity matrix were eluted from the affinity matrix and subsequently separated by SDS-Polyacrylamide gel elecrophoresis and transferred to a blotting membrane. Individual kinases were detected with specific antibodies against GSK3alpha, GSK3beta and ITK (Figure 4A). The result shows that preincubation of the cell lysate with Bis VIII prevented binding of the target proteins GSK3alpha and GSK3beta to the kinobeads matrix in a dose dependent manner. Increasing concentrations of the kinase inhibitor Bis VIII specifically prevented binding of GSK3 alpha and GSK3beta to the kinobeads but not binding of ITK. For GSK3beta the signal was quantified and plotted against the Bis VIII concentration added to the cell lysate (Figure 4B).

For the quantitative detection of proteins by mass spectrometry proteins were eluted from the affinity matrix and subsequently separated by SDS-Polyacrylamide gel elecrophoresis. Suitable gel areas were cut out and subjected to in-gel proteolytic digestion with trypsin. Four tryptic digest samples (corresponding to three different Bis VIII concentrations in the lysate and one DMSO control) were labeled with iTRAQ reagents and the combined samples were analyzed in a single LC-MS/MS mass spectrometry analysis followed by peak quantification in the MS/MS spectrum (Ross et al., 2004. Multiplexed protein quantitation in Saccharomyces cerevisiae using amine-reactive isobaric tagging reagents. Mol. Cell. Proteomics 3(12): 1154-1169). The result shows that different levels of individual proteins were detected and relative intensity values were calculated (Table 21). Binding curves for individual kinases are shown (Figure 5). The relative intensity 50 (RI50) values representing the affinity of kinase-compound pairs are similar to IC50 values reported by kinase enzyme assays (Davies et al., 2000. Specificity and mechanism of action of some commonly used protein kinase inhibitors. Biochemical Journal 351(Pt 1): 95-105).

### 1. Cell culture

Jurkat cells (clone E6-1 from ATCC, number TIB-152) were grown in 1 litre Spinner flasks (Integra Biosciences, #182101) in suspension in RPMI 1640 medium (Invitrogen, #21875-034) supplemented with 10% Fetal Bovine Serum (Invitrogen) at a density between 0.15 x 10⁶ and 1.2 x 10⁶ cells/ml and harvested by centrifugation. Cell pellets were frozen in liquid nitrogen and subsequently stored at -80°C.

### 2. Preparation of cell lysates

Jurkat cells were homogenized in a Potter S homogenizer in lysis buffer: 50 mM Tris-HCl, 0.8% NP40, 5% glycerol, 150 mM NaCl, 1.5 mM MgCl2, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 10 times using a mechanized POTTER S, transferred to 50 ml falcon tubes, incubated for 30 minutes on ice and spun down for 10 minutes at 20,000 g at 4°C (10,000 rpm in Sorvall SLA600, precooled). The supernatant was transferred to an ultracentrifuge (UZ)-polycarbonate tube (Beckmann, 355654) and spun for 1 hour at 100.000 g at 4°C (33.500 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Preincubation of lysate with test compound

Aliqots of Jurkat lysate (10 mg protein) were incubated with different Bis VIII concentrations for one hour at 4°C (0.025 µM, 0.074 µM, 0.22 µM, 0.67 µM, 2.0 µM and 6.0 µM final concentration of Bis VIII). To this end Bis VIII solutions were prepared in 100% DMSO as solvent that corresponded to 200 fold desired final Bis VIII concentration (Bis VIII from Alexis Biochemicals cat. number ALX 270-056). Five µl of these solutions were added to 1 ml of lysate resulting in the indicated final concentrations. For a control experiment 5 µl of DMSO without Bis VIII were used.

### 4. Protein capturing with kinobeads

To each preincubated lysate sample 40 µl of kinobeads (see example 1) were added and incubated for one hour at 4°C. During the incubation the tubes were rotated on an end-over-end shaker (Roto Shake Genie, Scientific Industries Inc.). Beads were collected by centrifugation, transfered to Mobicol-columns (MoBiTech 10055) and washed with 10 ml 1xDP buffer containing 0.4% NP40 detergent, followed by a wash with 5 ml 1xDP buffer with 0.2 % NP40. To elute the bound proteins, 100 µl 2x SDS sample buffer was added, the column was heated for 30 minutes at 50°C and the eluate was transferred to a microfuge tube by centrifugation. Proteins were then separated by SDS-Polyacrylamide electrophoresis (SDS-PAGE). The composition and preparation of buffers is described in example 2.

### 5. Protein Detection by Western Blot Analysis

Western blots were performed according to standard procedures and developed with the ECL Western blotting detection system according to the instructions of the manufacturer (Amersham Biosciences, #RPN2106). The ECL Western blotting system from Amersham is a light emitting non-radioactive method for the detection of specific antigens, directly or indirectly with Horseradish Peroxidase (HRP) labeled antibodies.

The anti-Glycogen Synthase Kinase 3 beta (GSK3beta) antibody was used at a dilution of 1:1000 (rabbit polyclonal anti-GSK3β, Stressgen Bioreagents,Victoria, Canada, product number KAP-ST002). This antibody also recognizes GSK3alpha. The anti-ITK antibody was also used at a dilution of 1:1000 (rabbit polyclonal anti-ITK antibody, Upstate Lake Placid, NY, catalog number 06-546).

### 6. Protein Detection by Mass Spectrometry

### 6.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 minutes) and subsequently alkylated with 55 mM iodoacetamid (in 5 mM ammonium bicarbonate) at room temperature in the dark for 30 minutes. Reduced and alkylated proteins were digested in gel with porcine trypsin (Promega) at a protease concentration of 10 ng/µl in 5mM Triethylammonium hydrogencarbonate (TEAB). Digestion was allowed to proceed for 4 hours at 37°C and the reaction was subsequently stopped using 5 µl 5% formic acid.

### 6.2 Sample preparation prior to analysis by mass spectrometry

Gel plugs were extracted twice with 20 µl 1% formic acid in water and once with 20 µl 0.1 % formic acid, 60% acetonitrile in water and pooled with acidified digest supernatants. Samples were dried in a a vaccuum.

### 6.3 iTRAQ labeling of peptide extracts

The peptide extracts of samples treated with different concentrations of the test compound (0.074 µM, 0.22 µM and 0.67 µM Bis VIII) and the solvent control (0.5% DMSO) were treated with different isomers of the isobaric tagging reagent (iTRAQ Reagents Multiplex Kit, part number 4352135, Applied Biosystems, Foster City, CA, USA). The iTRAQ reagents are a set of multiplexed, amine-specific, stable isotope reagents that can label all peptides in up to four different biological samples enabling simultaneous identification and quantitation of peptides. The iTRAQ reagents were used according to instructions provided by the manufacturer.

The samples were resuspended in 10 µl 50 mM TEAB solution, pH 8.5 and 10 µl ethanol were added. The iTRAQ reagent was dissolved in 85 µl ethanol and 10 µl of reagent solution were added to the sample. The labeling reaction was performed at room temperature for one hour on a horizontal shaker and stopped by adding 10 µl of 10% formic acid in water. The four labeled sampled were then combined, dried in a vacuum centrifuge and resuspended in 10 µl of 0.1 % formic acid in water.

### 6.4 Mass spectrometric data acquisition

Peptide samples were injected into a nano LC system (CapLC, Waters or Ultimate, Dionex) which was directly coupled to a quadrupole TOF (QTOF2, QTOF Ultima, QTOF Micro, Micromass) or ion trap (LTQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Table 3: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 6.5 Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis.

### 6.6 Protein quantitation

Relative protein quantitation was performed using peak areas of iTRAQ reporter ion signals essentially as described by Ross and colleagues (Ross et al., 2004. Multiplexed protein quantitation in Saccharomyces cerevisiae using amine-reactive isobaric tagging reagents. Mol. Cell. Proteomics 3(12): 1154-1169).

### 6.7 Binding curves and determination of RI50 values

The Relative Intensity (RI) values for the identified kinases are shown in Table 21. The test compound Bis VIII was used at three different concentrations in the cell lysate and the RI values were normalized to the DMSO control. For selected kinases the RI values were plotted against the concentration of Bis VIII and curve fitting was performed using the Xlfit program (ID Busiess Solutions Ltd.) using a hyperbolic equilibrium model (Figure 5). The RI50 value corresponds to the test compound (Bis VIII) concentration at which the relative intensity of the MS signal for a kinase is 50% compared to the solvent (DMSO) control.

### Table 21: Proteins identified by mass spectrometry analysis

The test compound Bis VIII was used at three different concentrations in the lysate and the resulting RI values were normalized to the DMSO control which was set to 1.0. Relative Intensity values are listed. The Representative refers to the database accession number of the International Protein Index (IPI, European Bioinformatics Institute), a compilation of protein sequences derived from several high-quality sequence databases (including GenBank, EMBL, SwissProt, RefSeq, Ensembl). Kinase names are in accordance with the human kinase nomenclature (http://kinase.com) and Manning et al., 2002, Science 298, 1912-1934 as specified in supplementary material).

| | | | **Sample 1** | **Sample 2** | **Sample 3** | **Sample 4** |
|---|---|---|---|---|---|---|
| **Representative** | **Clustername** | **Kinase Name** | **DMSO** | **0.074 uM** | **0.22 uM** | **0.67 uM** |
| IPI00298977.4 | AAK1 | AAK1 | 1.00 | 0.82 | 0.90 | 0.75 |
| IPI00329488.4 | ABL2 | ARG | 1.00 | 0.96 | 0.97 | 0.91 |
| IPI00176642.3 | AURKB | AurB | 1.00 | 0.95 | 1.04 | 0.97 |
| CZB00000043.1 | ABI1 | Abl1 | 1.00 | 0.81 | 0.90 | 1.03 |
| IPI00306217.1 | BLK | BLK | 1.00 | 0.90 | 0.96 | 0.83 |
| IPI00430291.1 | CAMK2D | CaMK2d | 1.00 | 0.91 | 0.99 | 0.88 |
| IPI00169392.3 | CAMK2G | CaMK2g | 1.00 | 0.83 | 0.98 | 0.88 |
| IPI00031681.1 | CDK2 | CDK2 | 1.00 | 0.89 | 0.85 | 0.65 |
| IPI00023530.4 | CDK5 | CDK5 | 1.00 | 0.92 | 1.02 | 0.95 |
| IPI00000685.1 | CDK7 | CDK7 | 1.00 | 1.03 | 1.04 | 0.89 |
| IPI00013212.1 | CSK | CSK | 1.00 | 0.84 | 0.94 | 0.89 |
| IPI00183400.8 | CSNK1A1 | CK1a | 1.00 | 0.97 | 1.04 | 1.02 |
| IPI00465058.1 | CSNK1G1 | CK1g1 | 1.00 | 1.01 | 1.05 | 0.96 |
| IPI00219012.2 | FYN | FYN | 1.00 | 0.91 | 1.04 | 0.93 |
| IPI00298949.1 | GAK | GAK | 1.00 | 0.92 | 1.01 | 0.97 |
| **IPI00292228.1** | **GSK3A** | GSK3A | 1.00 | 0.50 | 0.29 | 0.20 |
| **IPI00216190.1** | **GSK3B** | GSK3B | 1.00 | 0.54 | 0.39 | 0.28 |
| IPI00004566.1 | ITK | ITK | 1.00 | 0.80 | 0.94 | 0.91 |
| IPI00515097.1 | LCK | LCK | 1.00 | 0.87 | 1.00 | 0.94 |
| IPI00003479.1 | MAPK1 | Erk2 | 1.00 | 0.89 | 1.04 | 0.98 |
| IPI00002857.1 | MAPK14 | p38a | 1.00 | 0.82 | 0.95 | 0.93 |
| IPI00024672.1 | MAPK8 | JNK1 | 1.00 | 0.91 | 1.02 | 0.83 |
| IPI00024673.1 | MAPK9 | JNK2 | 1.00 | 0.97 | 1.08 | 1.04 |
| IPI00012069.1 | NQO1 | | 1.00 | 0.84 | 0.99 | 0.87 |
| IPI00219129.7 | NQO2 | | 1.00 | 0.87 | 0.95 | 0.78 |
| IPI00410287.1 | PRKAA1 | AMPKa1 | 1.00 | 0.88 | 1.00 | 0.91 |
| IPI00220409.2 | PRKAB1 | | 1.00 | 0.91 | 0.94 | 0.79 |
| IPI00549328.2 | PRKAG1 | | 1.00 | 0.78 | 0.79 | 0.68 |
| **IPI00385449.3** | **PRKCA** | PKCa | 1.00 | 0.25 | 0.19 | 0.18 |
| **IPI00219628.1** | **PRKCB1** | PKCb | 1.00 | 0.29 | 0.23 | 0.17 |
| IPI00029702.1 | PTK2B | PYK2 | 1.00 | 0.82 | 0.90 | 0.74 |
| IPI00465291.3 | SNF1LK2 | QIK | 1.00 | 0.93 | 1.11 | 0.98 |
| IPI00479211.2 | SRC | SRC | 1.00 | 0.93 | 0.98 | 0.94 |
| IPI00298940.2 | STK6 | AurA | 1.00 | 0.90 | 1.03 | 0.87 |
| IPI00293613.1 | TBK1 | TBK1 | 1.00 | 1.05 | 1.13 | 1.14 |
| IPI00411818.3 | ULK3 | ULK3 | 1.00 | 0.95 | 0.97 | 1.15 |
| IPI00025830.1 | WEE1 | Wee1 | 1.00 | 1.21 | 1.15 | 1.14 |
| IPI00477734.1 | YES1 | YES | 1.00 | 0.82 | 0.92 | 0.87 |

The following pages 114 to 118 contain specific embodiments.
1. A method for the characterization of at least one enzyme, comprising the steps of
   a) providing a protein preparation containg the enzyme,
   b) contacting the protein preparation under essentially physiological conditions with at least one broad spectrum enzyme ligand immobilized on a solid support under conditions allowing the binding of the enzyme to said broad spectrum enzyme ligand,
   c) eluting the enzyme, and
   d) characterizing the eluted enzyme by mass spectrometry.
2. The method of 1, wherein the provision of a protein preparation in step a) includes the steps of harvesting at least one cell containing the enzyme and lysing the cell.
3. A method for the characterization of at least one enzyme, comprising the steps of:
   a) providing two aliquots comprising each at least one cell containing the enzyme,
   b) incubating one aliquot with a given compound,
   c) harvesting the cells,
   d) lysing the cells,
   e) contacting the cell lysates under essentially physiological conditions with at least one broad spectrum enzyme ligand immobilized on a solid support under conditions allowing the binding of the enzyme to said broad spectrum enzyme ligand,
   f) eluting the enzyme or enzymes, and
   g) characterizing the eluted enzyme or enzymes by mass spectrometry.
4. The method of 3, wherein by characterizing the enzyme it is determined whether the administration of the compound results in a differential expression or activation state of the enzyme.
5. A method for the characterization of at least one enzyme, comprising the steps of:
   a) providing two aliquots of a protein preparation containing the enzyme,
   b) contacting one aliquot under essentially physiological conditions with at least one broad spectrum enzyme ligand immobilized on a solid support under conditions allowing the binding of the enzyme to said broad spectrum enzyme ligand,
   c) contacting the other aliquot under essentially physiological conditions with at least one broad spectrum enzyme ligand immobilized on a solid support under conditions allowing the binding of the enzyme to said broad spectrum enzyme ligand and with a given compound,
   d) eluting the enzyme or enzymes, and
   e) characterizing the eluted enzyme or enzymes by mass spectrometry.
6. The method of 5, wherein the provision of a protein preparation in step a) includes the steps of harvesting at least one cell containing the enzyme and lysing the cell.
7. The method of any of 5 or 6, wherein a reduced detection of the enzyme in the aliquot incubated with the compound indicates that the enzyme is a direct target of the compound.
8. A method for the characterization of at least one enzyme-compound complex, comprising the steps of:
   a) providing a protein preparation containg the enzyme,
   b) contacting the protein preparation under essentially physiological conditions with at least one broad spectrum enzyme ligand immobilized on a solid support under conditions allowing the binding of the enzyme to said broad spectrum enzyme ligand,
   c) contacting the bound enzymes with a compound to release at least one bound enzyme, and
   d) characterizing the released enzyme or enzymes by mass spectrometry, or
   e) eluting the enzyme or enzymes from the ligand and characterizing the enzyme or enzymes by mass spectrometry, thereby identifying one or more binding partners of the compound.
9. The method of 8, wherein the provision of a protein preparation in step a) includes the steps of harvesting at least one cell containing the enzyme and lysing the cell.
10. The method of 9, performed as a medium or high throughput screening.
11. The method of any of 3 to 10, wherein said compound is selected from the group consisting of synthetic compounds, or organic synthetic drugs, more preferably small molecule organic drugs, and natural small molecule compounds.
12. The method any of 1 to 11, wherein the enzyme is selected from the group consisting of a kinase, a phosphatase, a protease, a phophodiesterase, a hydrogenase, a dehydrogenase, a ligase, an isomerase, a transferase, an acetylase, a deacetylase, a GTPase, a polymerase, a nuclease, and a helicase .
13. The method of any of 1 to 12, wherein the ligand binds to 10% to 50 %, preferably 30% to 50% of the enzymes of a given class of enzymes.
14. The method of any of 1 to 13, wherein the ligand is an inhibitor.
15. The method of 14, wherein the enzyme is a kinase and the ligand is selected from the group consisting of Bisindolylmaleimide VIII, Purvalanol B, CZC00007324 (linkable PD173955), and CZC00008004.
16. The method of any of 1 to 15, wherein the characterization of the enzyme is performed by characterizing coeluated binding partners of the enzyme, enzyme subunits or posttranslational modifications of the enzyme.
17. The method of any of 1 to 16, wherein the characterization is performed by the identification of proteotypic peptides of the enzyme or of the binding partner of the enzyme.
18. The method of 17, wherein the characterization is performed by comparing the proteotypic peptides obtained for the enzyme or the binding partner with known proteotypic peptides.
19. The method of any of 1 to 18, wherein the solid support is selected from the group consisting of agarose, modified agarose, sepharose beads (e.g. NHS-activated sepharose), latex, cellulose, and ferro- or ferrimagnetic particles.
20. The method of any of 1 to 19, wherein the broad spectrum enzyme ligand is covalently coupled to the solid support.
21. The method of any of 1 to 20, wherein 1 to 10 different ligands, preferably 1 to 6, more preferably 1 to 4 are used.
22. The method of any of 1 to 21, wherein, when more than one ligand is used, each ligand is present on a different solid support.
23. The method of any of 1 to 21, wherein, when more than one ligand is used, at least two different ligands are present on one solid support.
24. The method of any of 1 to 23, wherein by characterizing the enzyme or compound-enzyme complex the identity of all or parts of the members of an enzyme class in the cell is determined.
25. The method of any of 3 to 24, wherein the compound is different from the ligand.
26. The method of any of 1 to 25, wherein the binding between ligand and enzyme is a non-covalent binding.
27. A method for the production of a pharmaceutical composition, comprising the steps of:
   a) identifying an enzyme-compound comples according to any of 6 to 17, and
   b) formulating the compound to a pharmaceutical composition.
28. The method of 27, further comprising the step of modulating the binding affinity of the compound to the enzyme.
29. Use of at least one broad spectrum enzyme ligand immobilized on a solid support for the characterization of at least one enzyme or for the characterization of at least on enzyme-compound complex.

## Claims

1. A method for the characterization of at least one enzyme, comprising the steps of:
a) providing two aliquots comprising each at least one cell containing the enzyme,
b) incubating one aliquot with a given compound,
c) harvesting the cells,
d) lysing the cells,
e) contacting the cell lysates under essentially physiological conditions with at least one broad spectrum enzyme ligand immobilized on a solid support under conditions allowing the binding of the enzyme to said broad spectrum enzyme ligand,
f) eluting the enzyme or enzymes, and
g) characterizing the eluted enzyme or enzymes by quantitative mass spectrometry.

2. The method of claim 1, wherein by characterizing the enzyme it is determined whether the administration of the compound results in a differential expression or activation state of the enzyme.

3. A method for the characterization of at least one enzyme, comprising the steps of:
a) providing two aliquots of a protein preparation containing the enzyme,
b) contacting one aliquot under essentially physiological conditions with at least one broad spectrum enzyme ligand immobilized on a solid support under conditions allowing the binding of the enzyme to said broad spectrum enzyme ligand,
c) contacting the other aliquot under essentially physiological conditions with at least one broad spectrum enzyme ligand immobilized on a solid support under conditions allowing the binding of the enzyme to said broad spectrum enzyme ligand and with a given compound,
d) eluting the enzyme or enzymes, and
e) characterizing the eluted enzyme or enzymes by quantitative mass spectrometry.

4. The method of claim 3, wherein a reduced detection of the enzyme in the aliquot incubated with the compound indicates that the enzyme is a direct target of the compound.

5. A method for the characterization of at least one enzyme-compound complex, comprising the steps of:
a) providing a protein preparation containing the enzyme,
b) contacting the protein preparation under essentially physiological conditions with at least one broad spectrum enzyme ligand immobilized on a solid support under conditions allowing the binding of the enzyme to said broad spectrum enzyme ligand,
c) contacting the bound enzymes with a compound to release at least one bound enzyme, and
d) characterizing the released enzyme or enzymes by mass spectrometry, or
e) eluting the enzyme or enzymes from the ligand and characterizing the enzyme or enzymes by mass spectrometry, thereby identifying one or more binding partners of the compound.

6. The method any of claims 1 to 5, wherein the enzyme is selected from the group consisting of a kinase, a phosphatase, a protease, a phophodiesterase, a hydrogenase, a dehydrogenase, a ligase, an isomerase, a transferase, an acetylase, a deacetylase, a GTPase, a polymerase, a nuclease, and a helicase .

7. The method of any of claims 1 to 6, wherein the ligand binds to 10% to 50 %, preferably 30% to 50% of the enzymes of a given class of enzymes.

8. The method of any of claims 1 to 7, wherein the ligand is an inhibitor, preferably wherein the enzyme is a kinase and the ligand is selected from the group consisting of Bisindolylmaleimide VIII, Purvalanol B, CZC00007324 (linkable PD173955), and CZC00008004.

9. The method of any of claims 1 to 8, wherein the characterization of the enzyme is performed by characterizing coeluated binding partners of the enzyme, enzyme subunits or posttranslational modifications of the enzyme.

10. The method of any of claims 1 to 9, wherein the characterization is performed by the identification of proteotypic peptides of the enzyme or of the binding partner of the enzyme, preferably wherein the characterization is performed by comparing the proteotypic peptides obtained for the enzyme or the binding partner with known proteotypic peptides.

11. The method of any of claims 1 to 10, wherein 1 to 10 different ligands, preferably 1 to 6, more preferably 1 to 4 are used.

12. The method of any of claims 1 to 11, wherein, when more than one ligand is used, each ligand is present on a different solid support.

13. The method of any of claims 1 to 12, wherein, when more than one ligand is used, at least two different ligands are present on one solid support.

14. The method of any of claims 1 to 13, wherein by characterizing the enzyme or compound-enzyme complex the identity of all or parts of the members of an enzyme class in the cell is determined.

15. The method of any of claims 1 to 14, wherein the compound is different from the ligand.

16. The method of any of the preceding claims, wherein the broad spectrum ligand is able to bind to some, but not all enzymes present in the protein preparation.
